# EUROPEAN PATENT SPECIFICATION

(11) **EP 4 159 910 B1**
(45) Date of publication and mention of the grant of the patent: **15.10.2025**
(21) Application number: 21812499.8
(22) Date of filing: 26.05.2021
(51) Int. Cl.: D05B 11/00, D05B 75/00, D05B 27/10, D05B 35/04, D05B 39/00, D05B 69/14, A61L 2/10, A61L 2/24, A61L 2/26, B08B 5/02, D06B 1/02, D06B 13/00, D06B 21/02, D06B 23/02, D06G 1/00

(54) **AUTOMATIC MATTRESS STERILIZATION DEVICE**
AUTOMATISCHE MATRATZENSTERILISATIONSVORRICHTUNG
DISPOSITIF DE STÉRILISATION AUTOMATIQUE DE MATELAS

(30) Priority: 27.05.2020 KR 20200063938; 27.05.2020 KR 20200063939; 27.05.2020 KR 20200063940; 27.05.2020 KR 20200063941
(43) Date of publication of application: 05.04.2023
(73) Proprietor: Suh, Jin Won, Seongnam-si, Gyeonggi-do 13433 (KR)
(72) Inventor: Suh, Jin Won, Seongnam-si, Gyeonggi-do 13433 (KR)
(74) Representative: AWA Sweden AB
(86) International application number: PCT/KR2021/006547
(87) International publication number: WO 2021/242009

(56) References cited:
- CN-U- 206 910 531
- CN-U- 210 228 010
- JP-A- 2001 000 513
- KR-A- 20080 087 339
- KR-A- 20100 100 074
- KR-A- 20130 119 097
- KR-A- 20130 119 097
- KR-A- 20150 012 960
- KR-B1- 101 133 980
- KR-U- 20120 002 319
- KR-U- 20170 000 144
- RU-C1- 2 083 226

## Description

### Technical Field

The present disclosure relates to an automatic mattress sterilization apparatus, and more particularly, to an automatic mattress sterilization apparatus capable of sterilizing a mattress while moving the mattress from one side of a sterilization part having an ultraviolet lamp to the other side thereof using a frame provided with a moving roller.

### Background Art

Mattresses of beds have a spring structure mounted therein, and are installed and used in a house of a user after being manufactured. Referring to a process of manufacturing the mattresses, the manufacture is composed of various processes such as a sheet sewing operation, a mattress sealing operation, and the like.

When the mattress is manufactured through various operations as described above, the mattress may be contaminated by various factors in the manufacturing process. In order to provide the mattress to a user, the mattress contaminated by various factors should be sterilized and then provided to the user.

However, since a mattress has a large volume and a significant weight, a sterilization operation for removing a contamination source of the mattress is difficult to be performed after the mattress is installed in a house of a user, and thus, the mattress should be finally sterilized before being supplied to the user.

However, in a conventional mattress manufacturing apparatus, a separate apparatus for sterilizing a mattress is not provided, and thus, the mattress is supplied to a user without being properly sterilized.

In KR20080087339A, a movable device for cleaning a mattress is provided to sanitize the mattress by consecutively vibrating the mattress, injecting air to the mattress using an air injector, sucking dusts from the mattress using a dust inhaler, and sterilizing and drying the mattress. A movable device for cleansing a mattress(1) comprises a perforated conveyor(2), a position sensor, a vibrator(30), an air injector, a dust inhaler(40), a sterilizer(50), and a dryer(54). The conveyor is installed in a cleaning space(11) isolated from outside, and transfers the mattress by repeatedly rotating forward and reversely using a drive part powered by an external power supply. The position sensor is connected to the drive part and installed in the cleaning space, senses the position of the mattress to control operational direction of the drive part. The vibrator vibrates the mattress, which is transferred along the conveyor, to eliminate dust. The air injector injects compressed air to the mattress to blow off dust. The dust inhaler is arranged close to the vibrator and the air injector to suck up the dust separated from the mattress. The sterilizer sterilizes the mattress and the dryer dries the mattress.

### Description of Embodiments

### Technical Problem

The present disclosure is directed to solving the problems described above, and more particularly, relates to an automatic mattress sterilization apparatus capable of sterilizing a mattress while moving the mattress from one side of a sterilization part to the other side thereof using a frame provided with a moving according to present claim 1

### Technical Solution to Problem

One aspect of the present disclosure provides an automatic mattress sterilization apparatus configured to sterilize a mattress, including a frame extending in one direction and on which the mattress is placed, a sterilization part coupled to an upper portion of the frame and having an inner space through which the mattress passes, and a moving part provided in the frame and configured to move the mattress placed on the frame, wherein the mattress is moved from one side of the sterilization part to the other side of the sterilization part via the inner space of the sterilization part by the moving part.

### Advantageous Effects of Disclosure

The present disclosure relates to an automatic mattress sterilization apparatus, and it is possible to sterilize a mattress by moving the mattress from one side of a sterilization part having an ultraviolet lamp to the other side thereof using a frame provided with a moving roller.

Further, according to the present disclosure, a contamination source of a mattress can be removed by controlling the operation of a moving part provided in a frame or applying vibration to the mattress through a vibration application part, and the contamination source attached to the mattress can be removed through a gas spraying part provided above the mattress.

### Brief Description of Drawings

FIG. 1 is a view illustrating an overall shape of an automatic mattress sterilization apparatus according to an embodiment of the present disclosure.
FIG. 2 is a view illustrating a sterilization part having an ultraviolet lamp according to an embodiment of the present disclosure.
FIG. 3 is a view illustrating that vibration is applied to a mattress by controlling the rotation of a moving roller provided in a frame through a control part according to an embodiment of the present disclosure.
FIG. 4 is a view illustrating that a first sensor part and a second sensor part are provided in a sterilization part according to an embodiment of the present disclosure.
FIG. 5 is an operation relationship view illustrating that opening and closing of an inlet part and an outlet part is controlled through a control part, a first sensor part, and a second sensor part according to an embodiment of the present disclosure.
FIG. 6 is a view illustrating a frame of a mattress edge sewing apparatus having a rotation driving part according to an embodiment of the present disclosure.
FIG. 7 is a perspective view of the mattress edge sewing apparatus having a rotation driving part according to an embodiment of the present disclosure.
FIG. 8 is a view illustrating that the rotation driving part is rotated according to an embodiment of the present disclosure.
FIG. 9 is a view illustrating a fixing bar capable of being raised or lowered with respect to the rotation driving part according to an embodiment of the present disclosure.
FIG. 10 is a view illustrating that a second conveyor belt and a third conveyor belt move in opposite directions according to an embodiment of the present disclosure.
FIG. 11 is a view illustrating a turnover part of the mattress edge sewing apparatus having a rotation driving part according to an embodiment of the present disclosure.
FIG. 12 is a view illustrating that a sheet is placed on a sewing apparatus having a sheet support using a rotating ball according to an embodiment of the present disclosure.
FIG. 13 is a view illustrating the sewing apparatus having a sheet support using a rotating ball according to an embodiment of the present disclosure.
FIG. 14 is a view illustrating the rotating ball and an insertion groove of a frame according to an embodiment of the present disclosure.
FIG. 15A is a view illustrating that a roller is provided between the rotating ball and an inner circumferential surface of the insertion groove according to an embodiment of the present disclosure, and FIG. 15B is a view illustrating that a spring is provided between the rotating ball and the inner circumferential surface of the insertion groove according to an embodiment of the present disclosure.
FIGS. 16A and 16B are views illustrating the rotating ball installed to be raisable or lowerable according to an embodiment of the present disclosure.
FIG. 17 is a view illustrating an operation relationship of the sewing apparatus having a sheet support using a rotating ball according to an embodiment of the present disclosure.
FIG. 18 is a view illustrating that a sensor part is provided in the sewing apparatus having a sheet support using a rotating ball according to an embodiment of the present disclosure.
FIG. 19 is a view illustrating that an alignment bar is provided in the sewing apparatus having a sheet support using a rotating ball according to an embodiment of the present disclosure.
FIGS. 20A and 20B are views illustrating the alignment bar, which is raisable or lowerable, according to an embodiment of the present disclosure.
FIG. 21 is a view illustrating that a sheet is placed on a sewing apparatus having a sheet support using an air compressor according to an embodiment of the present disclosure.
FIG. 22 is a view illustrating the sewing apparatus having a sheet support using an air compressor according to an embodiment of the present disclosure.
FIG. 23 is a view illustrating an operation relationship of the sewing apparatus having a sheet support using an air compressor according to an embodiment of the present disclosure.
FIG. 24 is a view illustrating that a sensor part is provided in the sewing apparatus having a sheet support using an air compressor according to an embodiment of the present disclosure.
FIG. 25 is a view illustrating that an alignment bar is provided in the sewing apparatus having a sheet support using an air compressor according to an embodiment of the present disclosure.
FIGS. 26A and 26B are views illustrating an alignment bar that is raisable or lowerable according to an embodiment of the present disclosure.

### Best Mode

An automatic mattress sterilization apparatus according to the present disclosure for solving the above-described problems includes a frame extending in one direction and on which the mattress is placed, a sterilization part coupled to an upper portion of the frame and having an inner space through which the mattress passes, and a moving part provided in the frame and configured to move the mattress placed on the frame, wherein the mattress may be moved from one side of the sterilization part to the other side of the sterilization part via the inner space of the sterilization part by the moving part.

In the automatic mattress sterilization apparatus according to the present disclosure for solving the above-described problems, an ultraviolet lamp configured to sterilize the mattress may be provided inside the sterilization part.

In the automatic mattress sterilization apparatus according to the present disclosure for solving the above-described problems, the moving part may include a plurality of moving rollers coupled to the frame.

The automatic mattress sterilization apparatus according to the present disclosure for solving the above-described problems further includes a control part configured to control a rotation of each of the moving rollers, wherein the control part controls the moving roller to rotate repeatedly in forward and reverse directions so that vibration is applied to the mattress, when the mattress is placed inside the sterilization part.

In the automatic mattress sterilization apparatus according to the present disclosure for solving the above-described problems, the control part controls the moving roller to repeatedly rotate in the forward and reverse directions, and the number of forward rotations of the moving roller is greater than the number of reverse rotations of the moving roller.

In the automatic mattress sterilization apparatus according to the present disclosure for solving the above-described problems, a gas spraying part configured to spray high-pressure gas onto the mattress may be provided in the sterilization part.

In the automatic mattress sterilization apparatus according to the present disclosure for solving the above-described problems, one side and the other side of the inner space may communicate with the outside so that the mattress is moved therethrough, an inlet part configured to open and close one side of the inner space may be provided at one side of the inner space, and an outlet part configured to open and close the other side of the inner space may be provided at the other side of the inner space.

In the automatic mattress sterilization apparatus according to the present disclosure for solving the above-described problems, a first sensor part configured to detect a distance between the inlet part of the sterilization part and one end of the mattress may be provided at one side of the sterilization part, and a second sensor part configured to detect a distance between one end of the mattress moving in the inner space and the outlet part of the sterilization part may be provided inside the sterilization part.

The automatic mattress sterilization apparatus according to the present disclosure for solving the above-described problems may further include a control part configured to control the opening and closing of each of the inlet part and the outlet part, wherein the control part may control each of the inlet part and the outlet part to be open when the distance detected by each of the first sensor part and the second sensor part is less than or equal to a designated value.

In the automatic mattress sterilization apparatus according to the present disclosure for solving the above-described problems, a vibration application part configured to apply vibration to the mattress may be provided in the sterilization part.

A mattress edge sewing apparatus having a rotation driving part according to the present disclosure for solving the above-described problems includes a frame formed in a rectangular plate shape and on which a mattress is placed, a processing part disposed on a side surface of the frame and configured to process edges of the mattress, a first shaft configured to divide the frame into frames while extending in a longitudinal direction, a second shaft configured to divide one side of the frame, which is obtained by dividing the frame through the first shaft, while extending in a transverse direction, a third shaft configured to divide the other side of the frame, which is obtained by dividing the frame through the first shaft, while extending in the transverse direction, and a conveyor belt is provided in each of the divided frames and configured to move in a direction parallel to the transverse direction.

In the mattress edge sewing apparatus having a rotation driving part according to the present disclosure for solving the above-described problems, the second shaft and the third shaft may not be disposed on the same line.

In the mattress edge sewing apparatus having a rotation driving part according to the present disclosure for solving the above-described problems, the second shaft may horizontally divide one side of the frame, which is obtained by dividing the frame through the first shaft, equally, and the third shaft may horizontally divide the other side of the frame, which is obtained by dividing the frame through the first shaft, unequally.

In the mattress edge sewing apparatus having a rotation driving part according to the present disclosure for solving the above-described problems, the frame may include a first frame disposed on one side of the first shaft and on an upper side of the second shaft, a second frame disposed on the other side of the first shaft and on an upper side of the third shaft, a third frame disposed on the other side of the first shaft and on a lower side of the third shaft, and a fourth frame disposed on one side of the first shaft and on a lower side the second shaft, the conveyor belt may include a first conveyor belt installed in the first frame, a second conveyor belt installed in the second frame, a third conveyor belt installed in the third frame, and a fourth conveyor belt installed in the fourth frame, the mattress edge sewing apparatus may further include a control part configured to control the movement of each of the first conveyor belt, the second conveyor belt, the third conveyor belt, and the fourth conveyor belt.

In the mattress edge sewing apparatus having a rotation driving part according to the present disclosure for solving the above-described problems, a rotation driving part formed in a bar shape may be installed on a side portion of the frame, and the rotation driving part may have one side coupled to the side portion of the frame and may rotate around the coupled one side in a direction toward inside the frame from the side portion of the frame.

In the mattress edge sewing apparatus having a rotation driving part according to the present disclosure for solving the above-described problems, a fixing portion configured to rotate together with the rotation driving part while being raisable and lowerable with respect to the rotation driving part may be provided on an upper portion of the rotation driving part.

In the mattress edge sewing apparatus having a rotation driving part according to the present disclosure for solving the above-described problems, the control part may control the second conveyor belt and the third conveyor belt such that the second conveyor belt and the third conveyor belt move in opposite directions when the rotation driving part rotates.

In the mattress edge sewing apparatus having a rotation driving part according to the present disclosure for solving the above-described problems, the control part may control the second conveyor belt and the third conveyor belt such that the second conveyor belt and the third conveyor belt move at the same speed.

In the mattress edge sewing apparatus having a rotation driving part according to the present disclosure for solving the above-described problems, the other side of the frame may be divided by the third shaft so that an area of the third frame is larger than an area of the second frame.

In the mattress edge sewing apparatus having a rotation driving part according to the present disclosure for solving the above-described problems, a turnover part formed in a bar shape may be installed on the second shaft, and may have one side coupled to the second shaft, and the other side of the turnover part may rotate around the coupled one side toward the upper portion of the frame.

A sewing apparatus having a sheet support using a rotating ball according to the present disclosure for solving the above-described problems includes a frame on which a sheet is placed, a processing part disposed on one side of the frame and configured to process an edge of the sheet, and a rotating ball that is inserted in an insertion groove, which is cut in an inner direction of the frame, has a portion protruding above the frame, and is rotatable, wherein a plurality of rotating balls are formed to be spaced apart from each other on the frame.

In the sewing apparatus having a sheet support using a rotating ball according to the present disclosure for solving the above-described problems, the rotating ball may be formed in a spherical shape, and the insertion groove may be formed in a spherical shape whose upper portion is partially cut away.

In the sewing apparatus having a sheet support using a rotating ball according to the present disclosure for solving the above-described problems, a roller in contact with the rotating ball may be provided on an inner circumferential surface of the insertion groove.

In the sewing apparatus having a sheet support using a rotating ball according to the present disclosure for solving the above-described problems, a spring may be provided between the insertion groove and the rotating ball.

In the sewing apparatus having a sheet support using a rotating ball according to the present disclosure for solving the above-described problems, the rotating ball may be installed to be raisable and lowerable inside the insertion groove, a portion of the rotating ball may not protrude above the frame when the rotating ball is lowered, and a portion of the rotating ball may protrude above the frame when the rotating ball is raised.

The sewing apparatus having a sheet support using a rotating ball according to the present disclosure for solving the above-described problems may further include a switch configured to adjust the raising or lowering of the rotating ball.

The sewing apparatus having a sheet support using a rotating ball according to the present disclosure for solving the above-described problems may further include a control part configured to control the raising or lowering of the rotating ball, wherein the control part may control the rotating ball to be raised when the sheet rotates as the operation of the processing part is stopped.

In the sewing apparatus having a sheet support using a rotating ball according to the present disclosure for solving the above-described problems, an alignment bar configured to come into contact with one side of the sheet while protruding above the frame may be provided in the frame.

In the sewing apparatus having a sheet support using a rotating ball according to the present disclosure for solving the above-described problems, the alignment bar may be raisable or lowerable in an inner direction of the frame, so that the alignment bar protrudes above the frame or does not protrude above the frame.

In the sewing apparatus having a sheet support using a rotating ball according to the present disclosure for solving the above-described problems, the control part may control the alignment bar to be lowered in the inner direction of the frame when the rotating ball rotates.

In the sewing apparatus having a sheet support using a rotating ball according to the present disclosure for solving the above-described problems, the plurality of rotating balls formed on the frame may be spaced apart from each other at an interval of 30 cm to 40cm.

A sewing apparatus having a sheet support using an air compressor according to the present disclosure for solving the above-described problems includes a frame on which a sheet is placed, and a processing part disposed on one side of the frame and configured to process an edge of the sheet, wherein an air compressor configured to spray air into the frame is provided on a lower portion of the frame, and a plurality of air spraying holes, each configured to discharge the air sprayed from the air compressor to the outside, are formed to be spaced apart each other in the frame.

The sewing apparatus having a sheet support using an air compressor according to the present disclosure for solving the above-described problems may further include a switch configured to control an operation of the air compressor.

The sewing apparatus having a sheet support using an air compressor according to the present disclosure for solving the above-described problems may further include a control part configured to control air spraying from the air compressor, wherein the control part may control the air compressor to spray air into the frame when the operation of the processing part is stopped and the sheet is rotated.

In the sewing apparatus having a sheet support using an air compressor according to the present disclosure for solving the above-described problems, a sensor part configured to detect whether the sheet is placed on the frame may be provided on an upper portion of the frame, and the control part may control the air compressor to spray air only when the sensor part detects that the sheet is placed on the frame.

In the sewing apparatus having a sheet support using an air compressor according to the present disclosure for solving the above-described problems, the control part may control the air compressor such that pressure of the air sprayed from each of the plurality of air spraying holes is different from each other.

In the sewing apparatus having a sheet support using an air compressor according to the present disclosure for solving the above-described problems, the sensor part detects a center portion of the sheet using a shape of the sheet, and the control part may control the air compressor to spray air so that pressure of the air sprayed from the air spraying hole disposed in a center portion of the sheet is greater than pressure of the air sprayed from the air spraying hole disposed in a periphery of the sheet.

In the sewing apparatus having a sheet support using an air compressor according to the present disclosure for solving the above-described problems, the plurality of air spraying holes formed on the frame may be spaced apart from each other at an interval of 40 cm to 50 cm.

In the sewing apparatus having a sheet support using an air compressor according to the present disclosure for solving the above-described problems, an alignment bar configured to come into contact with one side of the sheet while protruding above the frame may be provided in the frame.

In the sewing apparatus having a sheet support using an air compressor according to the present disclosure for solving the above-described problems, the alignment bar may be raisable or lowerable in an inner direction of the frame, so that the alignment bar protrudes above the frame or does not protrude above the frame.

In the sewing apparatus having a sheet support using an air compressor according to the present disclosure for solving the above-described problems, the control part may control the alignment bar to be lowered in the inner direction of the frame when the air compressor sprays air into the frame.

### Mode of Disclosure

Hereinafter, embodiments of the present disclosure will be described in detail with reference to the accompanying drawings. The embodiments of the present disclosure are provided to more completely explain the present disclosure to those having average skill in the art. Since the present disclosure is susceptible to various modifications and alternative forms, specific embodiments thereof are shown by way of example in the drawings and will herein be described in detail.

Similar reference numerals are used for similar components in describing each drawing. In the accompanying drawings, the dimensions of structures are illustrated to be enlarged or reduced compared to the actual size for clarity of the present disclosure.

The terminology used herein is for the purpose of describing particular embodiments only and is not intended to be limiting of the present disclosure. A singular expression includes a plural expression unless the context clearly indicates otherwise. It should be understood that the term such as "include" and "have" herein is intended to designate the presence of features, numbers, steps, operations, components, elements, or a combination thereof described in the specification and does not preclude the possibility of the presence or addition of one or more other features, numbers, steps, operations, components, elements, or combinations thereof.

Further, while terms such as "first," "second," and the like may be used to describe various components, such components should not be limited to the above terms. These terms are used only to distinguish one component from another component. For example, a first component may be called a second component, and similarly, a second component may be called a first component without departing from the scope of rights of the present disclosure.

When a component is referred to as being "connected or coupled to" another component, the component may be directly connected or coupled to the other component, but it should be understood that there may be a new component between the component and the other component. On the other hand, when a component is referred to as being "directly connected to" or "directly coupled to" another component, it should be understood that there is no new other component between the component and the another component.

Unless otherwise defined, all terms used herein, including technical or scientific terms, have the same meanings as those generally understood by those with ordinary knowledge in the field of art to which the present disclosure belongs. Such terms as those defined in a generally used dictionary are to be interpreted to have the meanings equal to the contextual meanings in the relevant field of art, and are not to be interpreted to have idealized or excessively formal meanings unless clearly defined in the present application.

The present disclosure relates to an automatic mattress sterilization apparatus, and relates to an automatic mattress sterilization apparatus capable of sterilizing a mattress while moving the mattress from one side of a sterilization part having an ultraviolet lamp to the other side thereof using a frame provided with a moving roller. Hereinafter, exemplary embodiments of the present disclosure will be described in detail with reference to the accompanying drawings

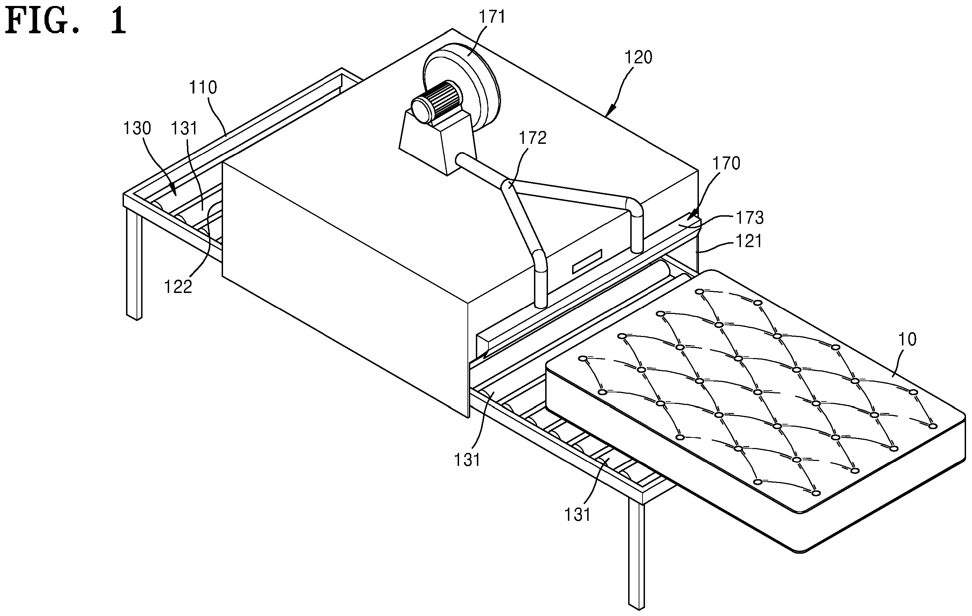

Referring to FIG. 1, an automatic mattress sterilization apparatus according to an embodiment of the present disclosure includes a frame 110, a sterilization part 120, and a moving part 130.

The frame 110 extends in one direction, and a mattress 10 is placed thereon. The frame 110 extends sufficiently long in the one direction so that the mattress 10 may be moved along the frame 110, and the mattress 10 is moved while being placed on an upper portion of the frame 110.

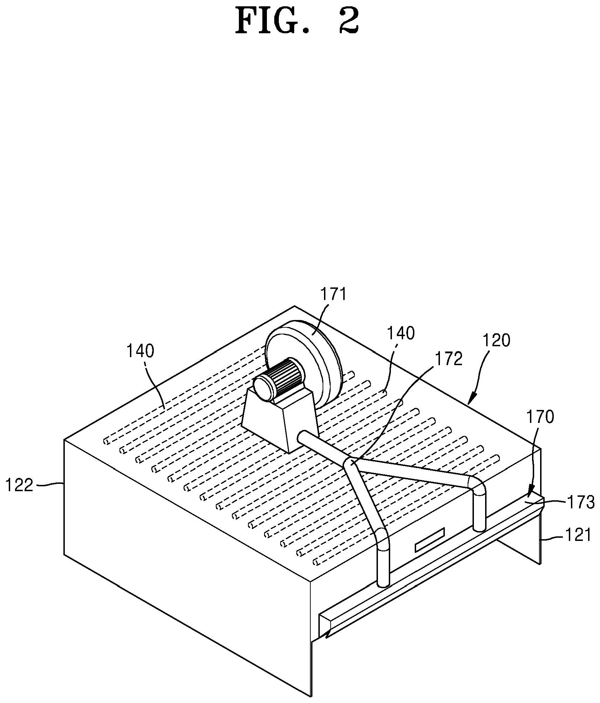

Referring to FIGS. 1 and 2, the sterilization part 120 is coupled to the upper portion of the frame 110 and has an inner space through which the mattress 10 may pass. The sterilization part 120 has the inner space through which the mattress 10 may pass, and one side and the other side of the inner space communicate with the outside.

The mattress 10 may enter the inner space of the sterilization part 120 through one side of the inner space, which communicates with the outside, and the mattress 10 may be discharged to the outside from the inner space of the sterilization part 120 through the other side of the inner space, which communicates with the outside.

More specifically, an inlet part 121 configured to open and close one side of the inner space is provided at one side of the inner space of the sterilization part 120, and an outlet part 122 configured to open and close the other side of the inner space may be provided at the other side of the inner space of the sterilization part 120.

As will be described below, the mattress 10 may be sterilized while passing through the sterilization part 120. Since the mattress 10 is sterilized through the sterilization part 120, an inside of the sterilization part 120 should be prevented from being contaminated by other contamination sources so that the mattress 10 may be cleanly sterilized through the sterilization part 120.

The inlet part 121 and the outlet part 122 are for preventing the inside of the sterilization part 120 from being contaminated, and the inlet part 121 and the outlet part 122 may open and close one side and the other side, respectively, of the inner space communicating with the outside.

Specifically, the sterilization part 120 prevented from being contaminated by allowing the inlet part 121 to be open only when the mattress 10 enters the sterilization part 120 and allowing the outlet part 122 to be open only when the mattress 10 is discharged from the sterilization part 120.

Referring to FIG. 2, an ultraviolet lamp 140 may be provided inside the sterilization part 120. A plurality of the ultraviolet lamps 140 may be provided inside the sterilization part 120, and the mattress 10 passing through the sterilization part 120 may be sterilized through the ultraviolet lamps 140.

The ultraviolet lamp 140 may be disposed at various points inside the sterilization part 120 as long as ultraviolet lamp 140 can sterilize the mattress 10 passing through the sterilization part 120.

The sterilization part 120 may sterilize the mattress 10 using the ultraviolet lamp 140, but the present disclosure is not limited thereto. Various devices for removing a contamination source of the mattress 10 may also be provided in the sterilization part 120 through which the mattress 10 passes.

According to an embodiment, a vibration application part (not shown) capable of applying vibration to the mattress 10 may also be provided in the sterilization part 120. Various configurations may be used for the vibration application part as long as vibration can be applied to the mattress 10, as the vibration is applied to the mattress 10 through the vibration application part, a contamination source such as dust attached to the mattress 10 may be removed.

The vibration application part may be a device that hits the mattress 10, and may also be a device that is connected to the mattress 10 and shakes the mattress 10. Various configurations may be used for the vibration application part as long as vibration can be applied to the mattress 10 to remove the contamination source of the mattress 10.

In the above description, the ultraviolet lamp 140 and the vibration application part are described as being provided in the sterilization part 120, but the present disclosure is not limited thereto. A device for sterilizing or cleaning the mattress 10 may be provided in the sterilization part 120, and it goes without saying that various devices may be provided as long as the contamination source of the mattress 10 can be removed.

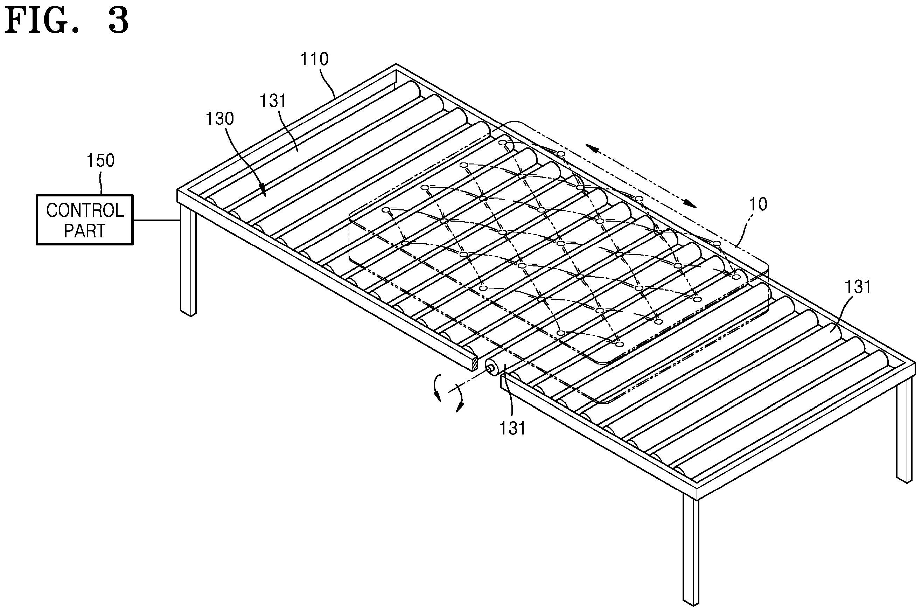

Referring to FIG. 3, the moving part 130 is provided in the frame 110, and may move the mattress 10 placed on the frame 110. The mattress 10 may be moved from one side of the sterilization part 120 to the inner space of the sterilization part 120 and then moved from the inner space of the sterilization part 120 to the other side of the sterilization part 120.

The moving part 130 may move the mattress 10, and various configurations may be used for the moving part 130 as long as they can move the mattress 10.

Referring to FIG. 3, the moving part 130 according to an embodiment may be a moving roller 131. A plurality of moving rollers 131 may be coupled to the frame 110, and a lower surface of the mattress 10 is brought into contact with the plurality of moving rollers 131.

When the moving roller 131 rotates after the lower surface of the mattress 10 is brought into contact with the moving roller 131, the mattress 10 may be moved by the rotation of the moving roller 131.

However, the moving part 130 is not limited to the moving roller 131, and may include various devices as long they may move the mattress 10.

The automatic mattress sterilization apparatus according to an embodiment of the present disclosure may further include a control part 150 configured to control the movement of the moving part 130.

When the mattress 10 is placed inside the sterilization part 120, the control part 150 may control the movement of the moving part 130 to apply vibration to the mattress 10.

Referring to FIG. 3, the control part 150 according to an embodiment of the present disclosure may control the rotation of the moving roller 131. When the mattress 10 is placed inside the sterilization part 120, the control part 150 may control the moving roller 131to rotate repeatedly in forward and reverse directions so that vibration is applied to the mattress 10.

The forward rotation of the moving roller 131 is a rotation for moving the mattress 10 from one side of the sterilization part 120 to the other side of the sterilization part 120, and the reverse rotation of the moving roller 131 is a rotation for moving the mattress 10 from the other side of the sterilization part 120 to one side of the sterilization part 120.

When the moving roller 131 repeatedly rotates in the forward and reverse directions by controlling the rotation of the moving roller 131 through the control part 150, the mattress 10 vibrates while repeatedly moving forward and backward inside the sterilization part 120.

When the mattress 10 is vibrated by the moving roller 131, a contamination source such as dust attached to the mattress 10 may be removed. Here, the control part 150 controls the moving roller 131 to repeatedly rotate in the forward and reverse directions, and the number of forward rotations of the moving roller 131 may be greater than the number of reverse rotations of the moving roller 131.

As described above, the mattress 10 is sterilized while being moved from one side of the sterilization part 120 to the other side thereof. Thus, in order to move the mattress 10 from one side of the sterilization part 120 to the other side thereof, the number of forward rotations of the moving roller 131 should be greater than the number of reverse rotations of the moving roller 131.

As described above, when the moving roller 131 repeatedly rotates in the forward and reverse directions through the control part 150, and the number of forward rotations of the moving roller 131 is greater than the number of reverse rotations of the moving roller 131, the mattress 10 may be moved from one side of the sterilization part 120 to the other side thereof while being vibrated.

The automatic mattress sterilization apparatus according to an embodiment of the present disclosure may further include a first sensor part 161 provided on one side of the sterilization part 120 and a second sensor part 162 provided inside the sterilization part 120.

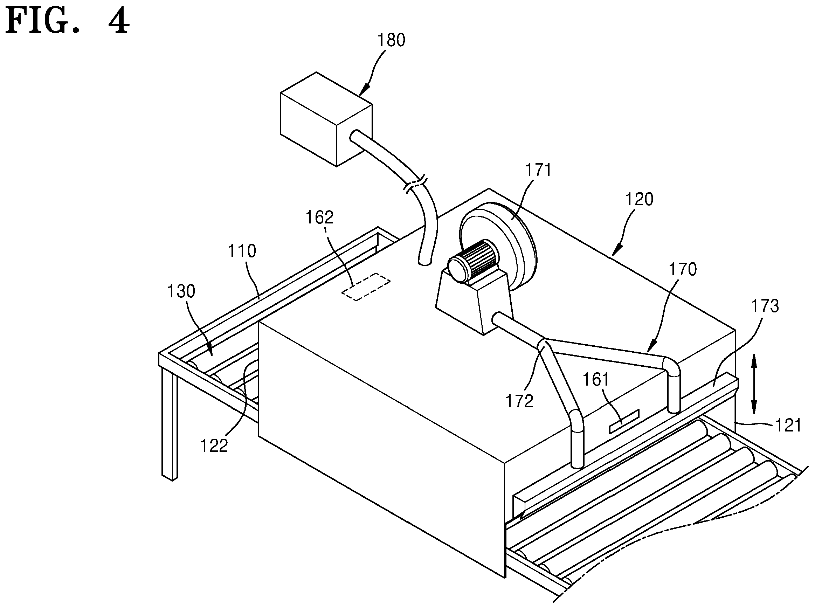

Referring to FIG. 4, the first sensor part 161 is provided at one side of the sterilization part 120, and may detect a distance between the inlet part 121 of the sterilization part 120 and one end of the mattress 10.

The second sensor part 162 is provided inside the sterilization part 120, and may detect a distance between the outlet part 122 of the sterilization part 120 and one end of the mattress 10.

As described above, the inlet part 121 and the outlet part 122 are provided in the sterilization part 120 in order to prevent the inner space from being contaminated. The inlet part 121 and the outlet part 122 should be opened when the mattress 10 approaches thereto, so that the mattress 10 may pass through the sterilization part 120 without being caught by the inlet part 121 and the outlet part 122.

The first sensor part 161 and the second sensor part 162 are provided for this purpose. The first sensor part 161 may recognize an approaching distance of the mattress 10 to the inlet part 121 of the sterilization part 120, and the second sensor part 162 may recognize an approaching distance of the mattress 10 to the outlet part 122 of the sterilization part 120.

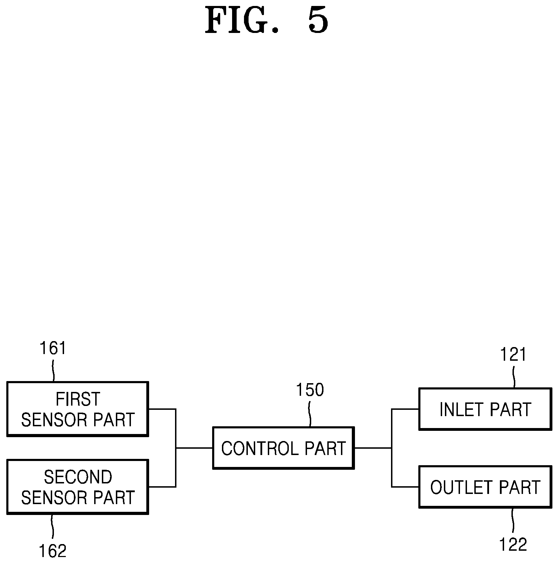

Referring to FIG. 5, the opening and closing of each of the inlet part 121 and the outlet part 122 may be controlled through the control part 150 capable of receiving signals of the first sensor part 161 and the second sensor part 162.

The control part 150 controls the inlet part 121 to be open and closed using the distance detected by the first sensor part 161, and when the distance detected by the first sensor part 161 is less than or equal to a designated value, the control part 150 controls the inlet part 121 to be open so that the mattress 10 may move into the sterilization part 120.

The control part 150 controls the outlet part 122 to be open and closed using the distance detected by the second sensor part 162, and when the distance detected by the second sensor part 162 is less than or equal to a designated value, the control part 150 controls the outlet part 122 to be open so that the mattress 10 may move from the inside of the sterilization part 120 to the outside.

The first sensor part 161 may be provided outside one side of the sterilization part 120 as the first sensor part 161 has to detect that the mattress 10 is approaching to the sterilization part 120, and the second sensor part 162 may be provided inside the sterilization part 120 as the first sensor part 161 has to detect that the mattress 10 is moving from the inside of the sterilization part 120 to the outside.

Here, the control part 150 may be the same device as the device configured to control the moving roller 131 or the movement of the moving part 130, but the present disclosure is not limited thereto. The control part 150 includes a first control part for controlling the movement of the moving part 130 or the moving roller 131, and a second control part for controlling the inlet part 121 and the outlet part 122. The first control part and the second control part may be integrally formed or may be formed separately.

Referring to FIG. 4, the sterilization part 120 of the automatic mattress sterilization apparatus according to an embodiment of the present disclosure may include a gas spraying part 170 configured to spray high-pressure gas onto the mattress 10.

The gas spraying part 170 may spray high-pressure gas onto the mattress 10 to remove a contamination source such as dust attached to the mattress 10. According to an embodiment, the gas spraying part 170 may be provided in the inlet part 121 of the sterilization part 120.

After the contamination source such as dust attached to the mattress 10 is removed through the gas spraying part 170 provided in the inlet part 121, the mattress 10 may enter inside the sterilization part 120.

However, the position of the gas spraying part 170 is not limited thereto, and the gas spraying part 170 may be provided at various points of the sterilization part 120 as long as a contamination source such as dust attached to the mattress 10 can be removed. For example, the gas spraying part 170 may be provided inside the sterilization part 120.

Various devices may be used for the gas spraying part 170 as long as they can spray high-pressure gas onto the mattress 10. According to an embodiment, the gas spraying part 170 may include a gas supply part 171 configured to supply high-pressure gas, a gas moving pipe 172 through which the high-pressure gas moves, and a gas spraying bar 173 from which the high-pressure gas is sprayed.

The gas supply part 171 generates high-pressure gas, and the gas generated by the gas supply part 171 may be moved to the gas spraying bar 173 via the gas moving pipe 172.

The gas moving pipe 172 may have a shape in which one pipe connected to the gas supply part 171 is branched into two, and the two branched pipes may be connected to the gas spraying bar 173.

The gas spraying bar 173 is formed in a bar shape while extending in a width direction, and the gas spraying bar 173 may spray the high-pressure gas along the width direction. Here, the width direction may be a direction perpendicular to a direction in which the mattress 10 moves or enters.

When the high-pressure gas is sprayed onto the mattress 10 along the width direction through the gas spraying bar 173, the high-pressure gas may be evenly sprayed on the entire area of the mattress 10 (the high-pressure gas may be sprayed onto the entire area of the mattress 10 while the mattress 10 passes through below the gas spraying part 170).

Referring to FIG. 4, the sterilization part 120 of the automatic mattress sterilization apparatus according to an embodiment of the present disclosure may include a gas discharge part 180 capable of discharging gas inside the sterilization part 120.

The gas discharge part 180 is a pipe extending from the inside of the sterilization part 120 to the outside of the sterilization part 120, and air inside the sterilization part 120 may be circulated through the gas discharge part 180.

As described above, when the mattress 10 is sterilized or vibrated inside the sterilization part 120, the inside of the sterilization part 120 may be contaminated. The gas discharge part 180 is provided to prevent such a phenomenon.

As the gas inside the sterilization part 120 is discharged to the outside through the gas discharge part 180, the air inside the sterilization part 120 may be purified, and through this, the inside of the sterilization part 120 may be prevented from being contaminated.

Various devices may be used for the gas discharge part 180 as long as they can discharge the air inside the sterilization part 120 to the outside while allowing the inside and the outside of the sterilization part 120 to communicate with each other.

The above-described automatic mattress sterilization apparatus according to an embodiment of the present disclosure has the following effects.

In the automatic mattress sterilization apparatus according to an embodiment of the present disclosure, the mattress 10 can be sterilized while being moved from one side of the sterilization part 120, which includes the ultraviolet lamp 140, to the other side thereof using the frame 110 provided with the moving roller 131.

In addition, in the automatic mattress sterilization apparatus according to an embodiment of the present disclosure, a contamination source of the mattress 10 can be removed by applying vibration to the mattress through the vibration application part or controlling the operation of the moving part 130 or the moving roller 131 provided in the frame 110, and a contamination source such as dust attached to the mattress 10 can be removed through the gas spraying part 170 provided above the mattress 10.

Hereinafter, a mattress edge sewing apparatus having a rotation driving part according to an embodiment of the present disclosure will be described in detail with reference to the accompanying drawings.

The present disclosure relates to a mattress edge sewing apparatus having a rotation driving part, and relates to a mattress edge sewing apparatus having a rotation driving part, capable of processing edges of a mattress by easily rotating the mattress while pushing and rotating the mattress through the rotation driving part by adjusting directions of a conveyor belt provided in a frame.

The mattress processed through the mattress edge sewing apparatus having a rotation driving part according to an embodiment of the present disclosure may be a mattress of a bed, but is not limited thereto, and may be formed in various forms as long as edges thereof can be processed.

In addition, a processing operation performed by the mattress edge sewing apparatus having a rotation driving part according to an embodiment of the present disclosure may be a mattress sewing operation through a sewing device, but is not limited thereto, and may include various operations as long as they are operations to process edges of a mattress.

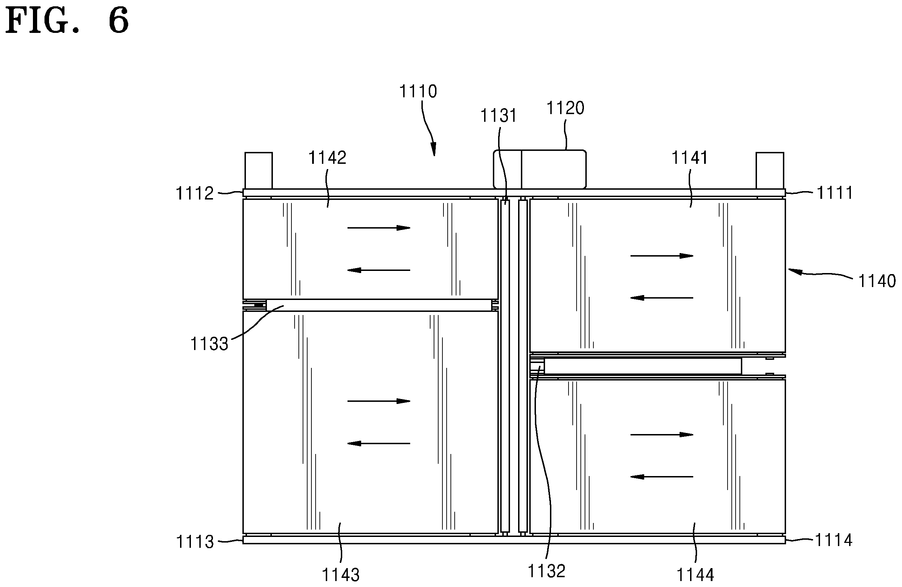

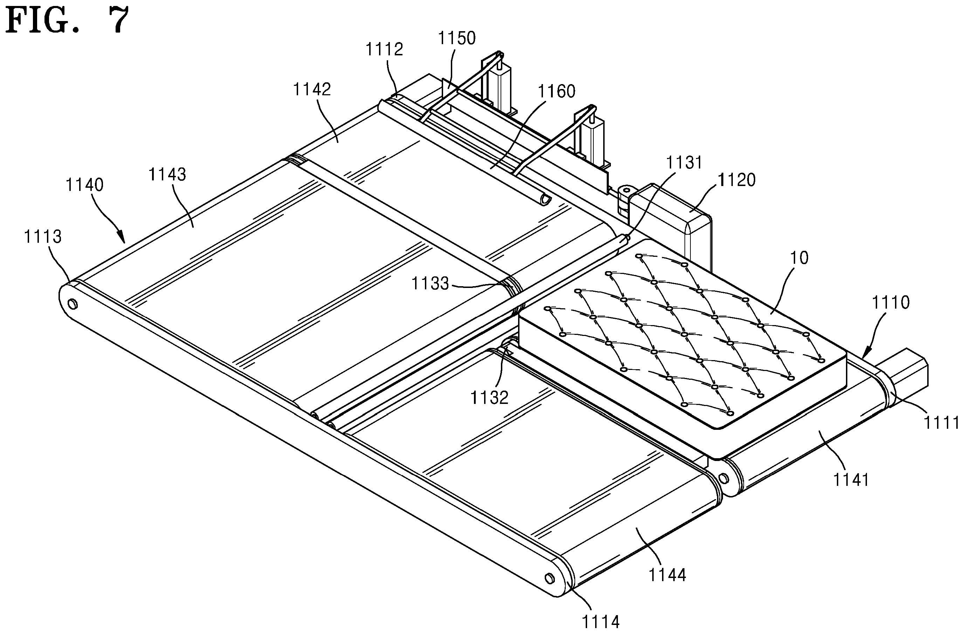

Referring to FIGS. 6 and 7, the mattress edge sewing apparatus having a rotation driving part according to an embodiment of the present disclosure includes a frame 1110, a processing part 1120, a first shaft 1131, a second shaft 1132, a third shaft 1133, and a conveyor belt 1140.

The frame 1110 may be formed in a rectangular plate shape, may be a work bench on which the mattress 10 is placed, and may be formed of a frame in which a plate is installed on an upper portion thereof so that edges of the mattress 10 are processed.

The processing part 1120 is disposed on a side surface of the frame 1110, and may process the edges of the mattress 10. The processing part 1120 may be a device capable of sewing the edges of the mattress 10. However, the processing part 1120 is not limited thereto, and the processing part 1120 may include various devices as long as they are devices capable of processing the edges of the mattress 10.

The edges of the mattress 10 may be processed while passing through the processing part 1120. The mattress 10 is formed in a rectangular shape. One edge of the mattress 10 passes through the processing part 1120, and then, the mattress 10 is rotated and the other edge of the mattress 10 is processed.

A conventional mattress processing apparatus does not have a configuration capable of rotating the mattress. Since the mattress has a large volume and a significant weight, through the conventional mattress processing apparatus, it is difficult to process the mattress while rotating the mattress.

In order to solve this problem, in the mattress edge sewing apparatus having a rotation driving part according to an embodiment of the present disclosure, the frame 1110 may be divided into a plurality of frames through the first shaft 1131, the second shaft 1132, and the third shaft 1133, and a rotation driving part 1150 may rotate the mattress while controlling a moving direction of the conveyor belt 1140 provided in the frame 1110.

Referring to FIG. 6, the first shaft 1131 divides the frame 1110 into frames while extending in a longitudinal direction. The first shaft 1131 vertically divides the frame 1110 into one side and the other side of the frame 1110.

Here, the longitudinal direction may be a vertical direction (in FIG. 6, a width direction in which the frame 1110 extends short) with respect to FIG. 6, and a transverse direction may be a horizontal direction (in FIG. 6, a width direction in which the frame 1110 extends long) with respect to FIG. 6. In addition, with respect to FIG. 6, one side of the frame 1110 may be a side of the first shaft 1131 in a rightward direction, and the other side of the frame 1110 may be a side of the first shaft 1131 in a leftward direction.

The second shaft 1132 divides one side of the frame 1110, which is obtained by dividing frame 1110 through the first shaft 1131, while extending in the transverse direction. The third shaft 1133 divides the other side of the frame 1110, which is obtained by dividing the frame 1110 through the first shaft 1131, while extending in the transverse direction.

That is, the second shaft 1132 horizontally divides one side of the frame 1110, which is obtained by dividing the frame 1110 through the first shaft 1131, and the third shaft 1133 horizontally divides the other side of the frame 1110, which is obtained by dividing the frame 1110 through the first shaft 1131.

Here, the second shaft 1132 and the third shaft 1133 may not be disposed on the same line. Referring to FIG. 6, one side and the other side of the frame 1110 may be divided such that the second shaft 1132 and the third shaft 1133 are not disposed on a single horizontal line.

Specifically, the second shaft 1132 may horizontally divide one side of the frame 1110, which is obtained by dividing the frame 1110 through the first shaft 1131, equally, and the third shaft 1133 may horizontally divide the other side of the frame 1110, which is obtained by dividing the frame 1110 through the first shaft 1131, unequally.

The mattress 10 may be moved while being placed on the frame 1110, and one side of the frame 1110 may be used as a point at which the mattress 10 is moved in the transverse direction, and thus the second shaft 1132 may horizontally divide one side of the frame 1110 into frames with equal areas.

In addition, as will be described below, since the other side of the frame 1110 may be used as a point at which the mattress 10 is rotated, the third shaft 1133 preferably horizontally divides the other side of the frame 1110 into frames with unequal areas.

The conveyor belt 1140 is provided in each of the divided frames 1110, and may move in a direction parallel to the transverse direction. The mattress 10 may be moved or rotated by the movement of the conveyor belt 1140 provided in the frame 1110, and the conveyor belt 1140 provided in each of the divided frames 1110 may be moved independently.

Specifically, the frame 1110 may be divided into a first frame 1111, a second frame 1112, a third frame 1113, and a fourth frame 1114, and the conveyor belt 1140 may include a first conveyor belt 1141, a second conveyor belt 1142, a third conveyor belt 1143, and a fourth conveyor belt 1144.

Referring to FIGS. 6 and 7, the first frame 1111 is disposed on one side of the first shaft 1131 and on an upper side of the second shaft 1132, and the second frame 1112 is disposed on the other side of the first shaft 1131 and on an upper side of the third shaft 1133.

The third frame 1113 is disposed on the other side of the first shaft 1131 and on a lower side of the third shaft 1133, and the fourth frame 1114 is disposed on one side of the first shaft 1131 and on a lower side the second shaft 1132.

The first conveyor belt 1141 may be provided in the first frame 1111 and may move forward or backward in a direction parallel to the transverse direction, and the second conveyor belt 1142 may be provided in the second frame 1112 and may move forward or backward in a direction parallel to the transverse direction.

The third conveyor belt 1143 is provided in the third frame 1113 and may move forward or backward in a direction parallel to the transverse direction, and the fourth conveyor belt 1144 is provided in the fourth frame 1114 and may move forward or backward in a direction parallel to the transverse direction.

The mattress edge sewing apparatus having a rotation driving part according to an embodiment of the present disclosure may further include the rotation driving part 1150 that is formed in a bar shape and capable of rotating the mattress 10.

The rotation driving part 1150 is formed in a bar shape and may rotate, and is installed on the side surface of the frame 1110 and may rotate toward the inside of the frame 1110.

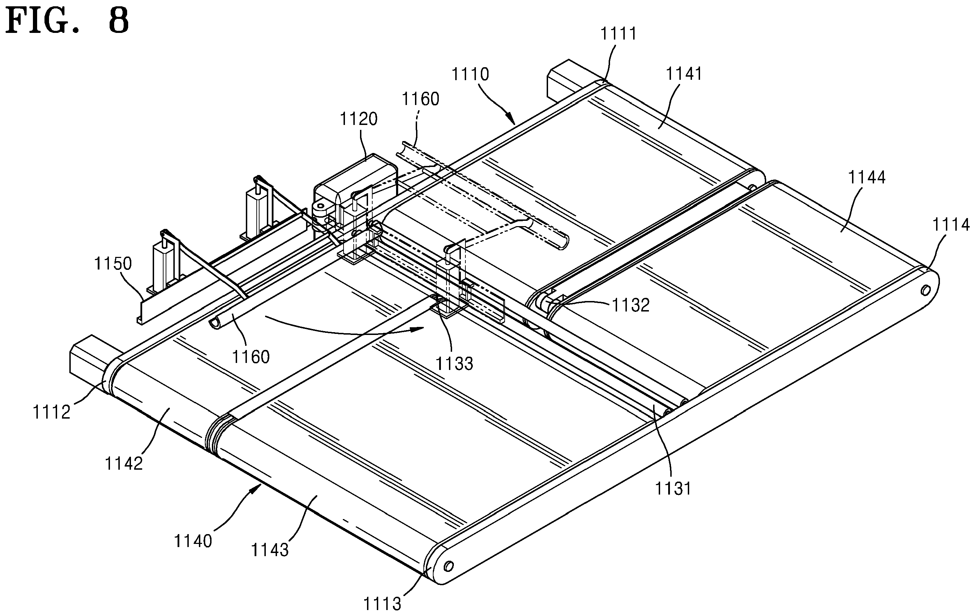

Referring to FIG. 8, as one side of the rotation driving part 1150 is coupled to a side portion of the frame 1110, the other side of the rotation driving part 1150 may rotate around the one side in a direction from the side portion of the frame 1110 toward the inside of the frame 1110.

Specifically, the rotation driving part 1150 may be installed on a side surface of the second frame 1112. One side of the rotation driving part 1150 is installed in the second frame 1112 at a point close to the first shaft 1131 and extends along the side surface of the second frame 1112.

The rotation driving part 1150 may rotate around one side thereof, and the rotation driving part 1150 may sweep an upper portion of the second frame 1112 while rotating.

The mattress 10 moved to the second frame 1112 may rotate by the operation of the rotation driving part 1150. Specifically, the edge of the mattress 10, which is processed through the processing part 1120, is brought into contact with the rotation driving part 1150 while being moved to the second frame 1112. When the rotation driving part 1150 rotates, the mattress 10 in contact with the rotation driving part 1150 rotates while being pushed.

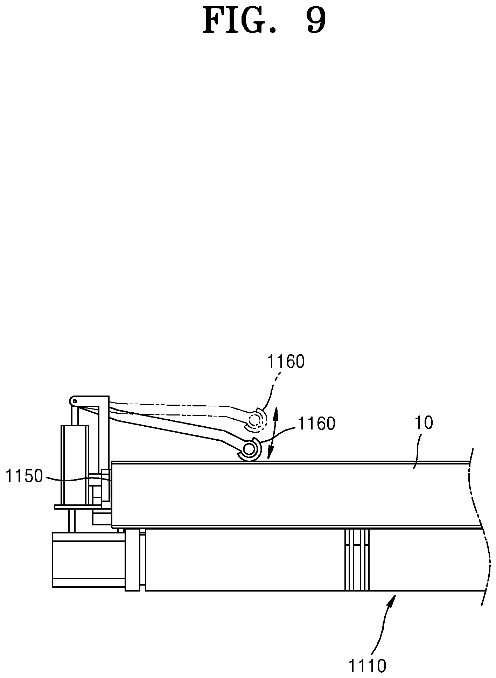

Referring to FIG. 9, the mattress edge sewing apparatus having a rotation driving part according to an embodiment of the present disclosure may further include a driving part 1160.

The driving part 1160 is provided on an upper portion of the rotation driving part 1150 and may rotate together with the rotation driving part 1150 while being raisable and lowerable with respect to the rotation driving part 1150.

The driving part 1160 fixes the mattress 10 when rotating the mattress 10 through the rotation driving part 1150. When the mattress 10 is not fixed when the mattress 10 is rotated by the rotation driving part 1150, there is a risk that the mattress 10 is not rotated in a target direction while being separated from the rotation driving part 1150.

The driving part 1160 is provided to prevent such a phenomenon, and the driving part 1160 may be formed in a bar shape or a rod shape. The driving part 1160 fixes an upper surface of the mattress 10 while moving downward before the rotation driving part 1150 rotates.

The driving part 1160 may rotate together with the rotation driving part 1150, and when the mattress 10 is fixed through the driving part 1160, the mattress 10 may be rotated while being prevented from being separated from the rotation driving part 1150.

The mattress edge sewing apparatus having a rotation driving part according to an embodiment of the present disclosure may further include a control part capable of controlling the movement of the conveyor belt 1140. The control part may control the movement of each of the first conveyor belt 1141, the second conveyor belt 1142, the third conveyor belt 1143, and the fourth conveyor belt 1144.

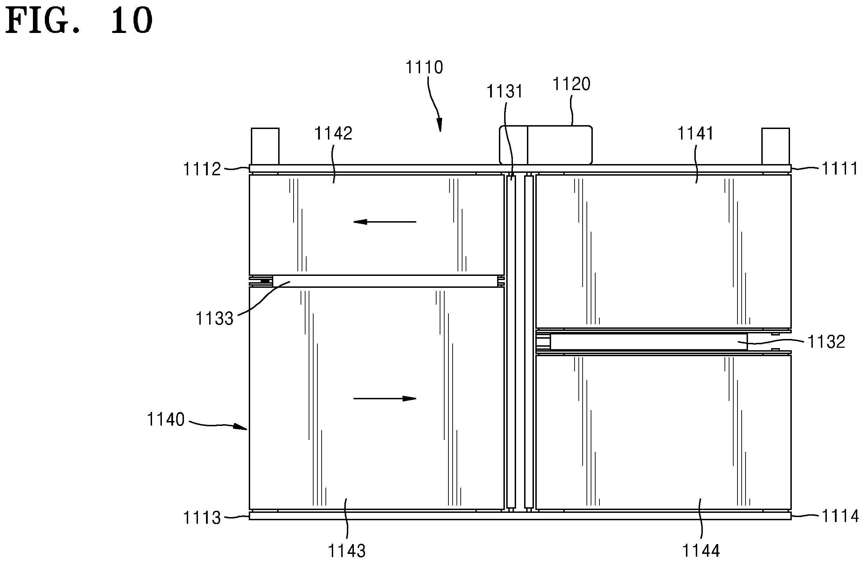

Referring to FIG. 10, when the mattress 10 placed on the second frame 1112 is rotated by the rotation driving part 1150, the control part may control the second conveyor belt 1142 and the third conveyor belt 1143 such that the second conveyor belt 1142 and the third conveyor belt 1143 move in opposite directions.

When the mattress 10 placed on the second frame 1112 is rotated by the rotation driving part 1150, there is a risk that the mattress 10 is not smoothly rotated due to a frictional force between the mattress 10 and the conveyor belt 1140.

In order to prevent such a phenomenon, the control part may control the second conveyor belt 1142 and the third conveyor belt 1143 such that the second conveyor belt 1142 and the third conveyor belt 1143 move in opposite directions.

Specifically, referring to FIG. 10, when the mattress 10 is rotated by the rotation driving part 1150, the upper portion (a portion coming in contact with the rotation driving part 1150) of the mattress 10 is rotated leftward with respect to FIG. 10, and a lower portion (a portion opposite to the portion coming in contact with the rotation driving part 1150) of the mattress 10 is rotated rightward with respect to FIG. 10.

Accordingly, the control part may control such that the second conveyor belt 1142 moves leftward with respect to FIG. 10 and the third conveyor belt 1143 moves rightward with respect to FIG. 10. The mattress 10 may be easily rotated by the above-described operation of the control part and the rotation driving part 1150.

Here, the other side of the frame 1110 may be divided by the third shaft 1133 so that an area of the third frame 1113 is larger than an area of the second frame 1112.

As described above, the third shaft 1133 may horizontally divide the other side of the frame 1110 unequally, and when the other side of the frame 1110 is divided through the third shaft 1133, the area of the third frame 1113 may be larger than the area of the second frame 1112.

Since the rotation driving part 1150 is provided on the side surface of the second frame 1112 and rotates, a rotation radius of the mattress 10 in the portion of the third frame 1113 is larger than the rotation radius of the mattress 10 in the portion of the second frame 1112.

That is, when the mattress 10 is rotated by the rotation driving part 1150, the mattress 10 in the portion of the third frame 1113 should be rotated with a larger radius. Accordingly, the mattress 10 may be easily rotated when the lower portion (a portion opposite to the portion coming in contact with the rotation driving part 1150) of the mattress 10 placed on the third frame 1113 receives a greater force.

To this end, the area of the third frame 1113 may be larger than the area of the second frame 1112, and as the area of the third frame 1113 is increased, a greater force may be provided to the lower portion of the mattress 10.

In addition, in order to rotate the mattress 10 in the target direction, the control part may control the second conveyor belt 1142 and the third conveyor belt 1143 such that the second conveyor belt 1142 and the third conveyor belt 1143 move at the same speed.

Since there is a risk that the mattress 10 is rotated in a direction different from the target direction when the second conveyor belt 1142 and the third conveyor belt 1143 do not move at the same speed, the control part may control the second conveyor belt 1142 and the third conveyor belt 1143 such that the second conveyor belt 1142 and the third conveyor belt 1143 move at the same speed.

However, the present disclosure is not limited thereto, and the control part may control the second conveyor belt 1142 and the third conveyor belt 1143 to move at different speeds depending on the size of the mattress 10 or the direction in which the mattress 10 rotates.

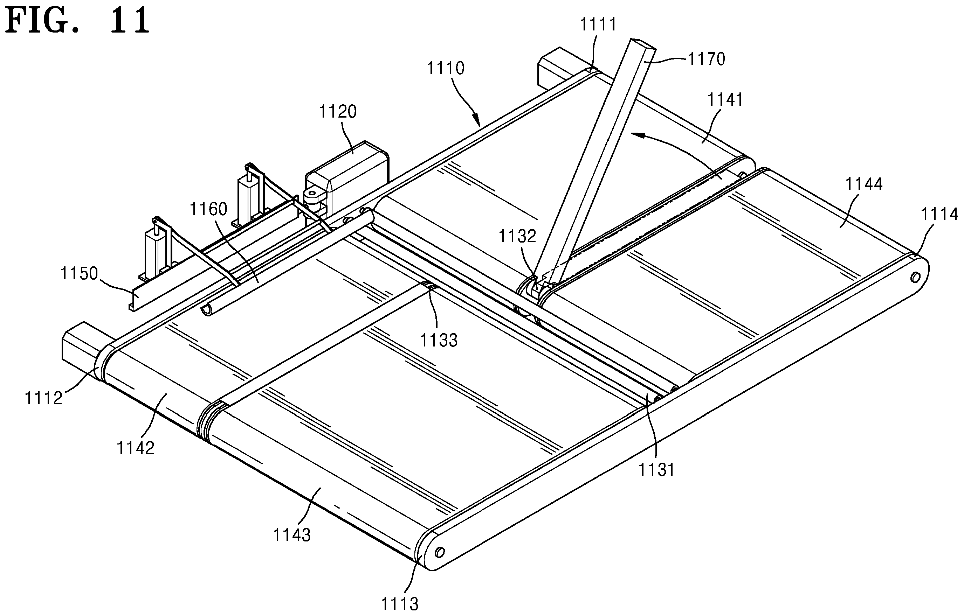

Referring to FIG. 11, the mattress edge sewing apparatus having a rotation driving part according to an embodiment of the present disclosure may further include a turnover part 1170 formed in a bar shape. The turnover part 1170 may be installed on the second shaft 1132, and has one side coupled to the second shaft 1132, and thus the other side of the turnover part 1170 may rotate toward the upper portion of the frame 1110 around the coupled one side.

The turnover part 1170 may be used to turn the mattress 10 over. The turnover part 1170 may be raised while rotating in an upward direction in which an upper surface of the frame 1110 faces. One side of the turnover part 1170 may be coupled to the second shaft 1132, and may be coupled to the second shaft 1132 at a position close to the first shaft 1131.

When the mattress 10 is placed on the first frame 1111 and the fourth frame 1114, the turnover part 1170 turns the mattress 10 over while rotating around one side thereof coupled to the second shaft 1132. The overturned mattress 10 is moved to the second frame 1112 and the third frame 1113.

The mattress edge sewing apparatus having a rotation driving part according to an embodiment of the present disclosure may operate as follows.

The mattress 10 is formed in a rectangular shape and has a predetermined thickness, and each of four edges of the mattress 10 should be sewn at the upper and lower surfaces of the mattress 10.

The mattress 10 is first placed on the first frame 1111 and the fourth frame 1114 and passes through the processing part 1120, thereby sewing one edge of the mattress 10.

The mattress 10 that has passed through the processing part 1120 is moved to the second frame 1112 and the third frame 1113, and the mattress 10 moved to the second frame 1112 and the third frame 1113 may be rotated by the rotation driving part 1150 and the driving part 1160.

In this case, the control part controls such that the second conveyor belt 1142 provided in the second frame 1112 and the third conveyor belt 1143 provided in the third frame 1113 move in opposite directions so that the mattress 10 rotates easily.

When the mattress 10 is rotated by the rotation driving part 1150, the conveyor belt 1140 is operated to place the mattress 10 again on the first frame 1111 and the fourth frame 1114. Thereafter, another edge of the mattress 10 is sewn. All of the four edges of the mattress 10 may be sewn while repeating the above-described process.

Since the mattress 10 has a certain thickness, both the upper and lower surfaces of the mattress 10 should be sewn in order to manufacture the mattress 10. After all of the four edges of the upper surface of the mattress 10 are completely sewn, the mattress 10 may be turned over by the turnover part 1170 so that the four edges of the lower surface of the lower surface of the mattress 10 are sewn.

Specifically, when the mattress 10 is placed on the first frame 1111 and the fourth frame 1114, the mattress 10 is turned over while the turnover part 1170 is rotated. The overturned mattress 10 is moved to the second frame 1112 and the third frame 1113, and the mattress 10 moved to the second frame 1112 and the third frame 1113 is moved again to the first frame 1111 and the fourth frame 1114 by the operation of the conveyor belt 1140.

When the upper and lower surfaces of the mattress 10 are turned over by the turnover part 1170, and then, the mattress 10 is placed on the first frame 1111 and the fourth frame 1114 and the above-described process is repeated, the four edges of the lower surface of the mattress 10 may be sewn.

The mattress edge sewing apparatus having a rotation driving part according to an embodiment of the present disclosure has the following effects.

In the mattress edge sewing apparatus having a rotation driving part according to an embodiment of the present disclosure, four edges of the mattress 10 can be processed while easily rotating the mattress 10 by pushing and rotating the mattress 10 through the rotation driving part 1150 while adjusting the directions of the conveyor belts 1140 provided in the frames 1110.

The mattress 10 according to an embodiment of the present disclosure is formed in a rectangular shape and has a predetermined thickness, and each of the four edges of the mattress 10 should be sewn at the upper and lower surfaces of the mattress 10.

In the mattress edge sewing apparatus having a rotation driving part according to an embodiment of the present disclosure, four edges of the upper surface of the mattress 10 can be processed while rotating the mattress 10 through the operation of the rotation driving part 1150 and the conveyor belt 1140. Thereafter, four edges of the lower surface of the mattress 10 can be processed as the mattress 10 is turned over by the turnover part 1170.

Hereinafter, a sewing apparatus having a sheet support using a rotating ball according to an embodiment of the present disclosure will be described in detail with reference to the accompanying drawings.

The present disclosure relates to a sewing apparatus having a sheet support using a rotating ball, and relates to a sewing apparatus having a sheet support using a rotating ball, capable of processing an edge of a sheet by easily rotating the sheet by providing a rotating ball in a frame of a processing device configured to process an edge of a sheet.

A sheet processed by the sewing apparatus having a sheet support using a rotating ball according to an embodiment of the present disclosure may be a sheet placed on the mattress, but the present disclosure is not limited thereto, and the sheet may be a mattress.

In addition, a processing operation performed in the sewing apparatus having a sheet support using a rotating ball according to an embodiment of the present disclosure may be a sewing operation through a sewing apparatus such as a sewing machine, but is not limited thereto, and may include various operations as long as they are operations that process an edge of a sheet or a mattress.

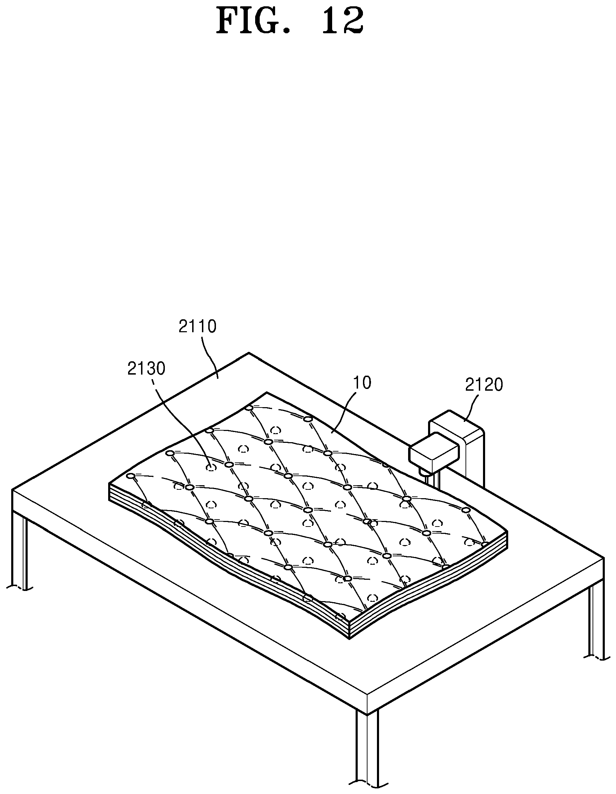

Referring to FIG. 12, the sewing apparatus having a sheet support using a rotating ball according to an embodiment of the present disclosure includes a frame 2110, a processing part 2120, and a rotating ball 2130.

The frame 2110 is a work bench on which a mattress 10 is placed, and may be formed of a frame in which a plate is installed in an upper portion thereof so that an edge of the mattress 10 may be processed thereon. The frame 2110 may be formed in various shapes as long as the mattress 10 is placed and processed thereon.

The processing part 2120 may be disposed on one side of the frame 2110, and may process an edge of the mattress 10. The processing part 2120 is a device capable of sewing an edge of the mattress 10, and may be a device such as a sewing machine.

However, the processing part 2120 is not limited to the sewing machine, and may include various devices as long as they are devices capable of processing an edge of the mattress 10. For example, the processing part 2120 may be a device configured to seal and sew an edge of the mattress 10.

An edge of the mattress 10 may be processed while passing through the processing part 2120. The mattress 10 is formed in a rectangular shape, and after one edge of the mattress 10 passes through the processing part 2120, the mattress 10 is rotated so that another edge of the mattress 10 is processed.

A configuration capable of rotating a sheet is not provided in a conventional sheet processing device. Since a sheet has a large volume and a significant weight, through the conventional sheet processing device, it is difficult to process the sheet while rotating the sheet.

In order to solve the above problem, the sewing apparatus having a sheet support using a rotating ball according to an embodiment of the present disclosure may use the rotating balls 2130.

The rotating ball 2130 is provided in the frame 2110, and an insertion groove 2111 for inserting the rotating ball 2130 therein is formed in the frame 2110. The rotating ball 2130 is inserted into the insertion groove 2111, and is rotatable as a portion thereof protrudes from an upper portion of the frame 2110.

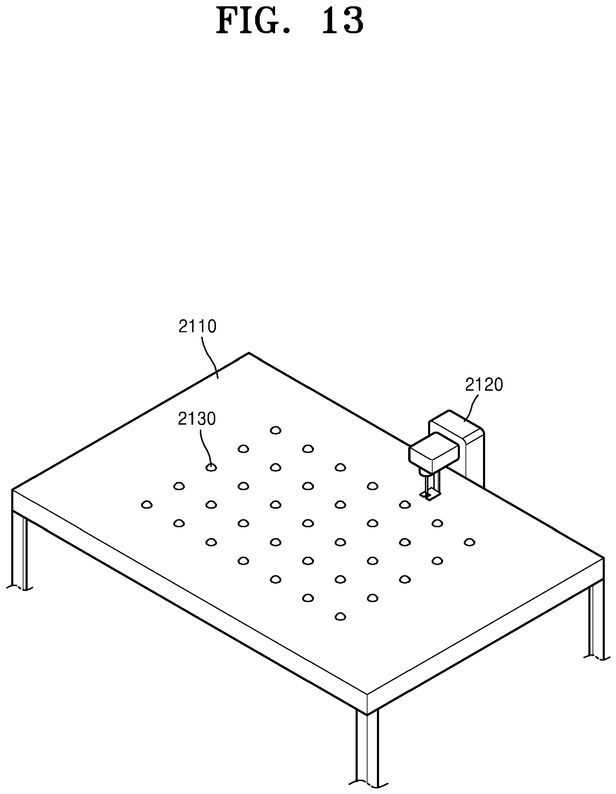

A plurality of rotating balls 2130 may be provided in the frame 2110, and the plurality of rotating balls 2130 may be formed to be spaced apart from each other. Referring to FIGS. 12 and 13, the mattress 10 is brought into contact with the rotating balls 2130 while being placed on the frame 2110, and as the rotating balls 2130 are rotatable, the mattress 10mattress 10 placed on the frame 2110 may be easily rotated.

In general, the mattress 10mattress 10 is difficult to rotate due to a frictional force generated between the mattress 10 and the frame 2110. However, when the rotatable rotating ball 2130 is used, the frictional force generated between the mattress 10 and the frame 2110 may be reduced, and as the rotating ball 2130 rotates, the mattress 10 may be easily rotated.

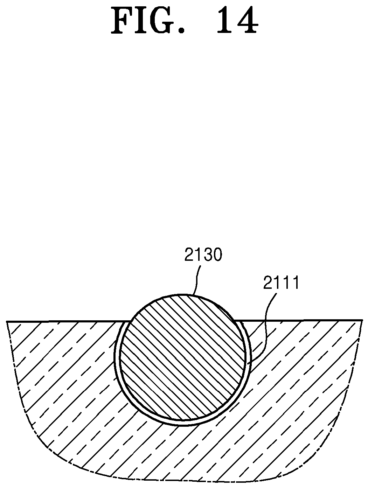

Referring to FIG. 14, the rotating ball 2130 may be formed in a spherical shape. The insertion groove 2111 may be formed in a spherical shape, and may be formed in a spherical shape whose upper portion is partially cut away.

An upper end of the rotating ball 2130 may be in contact with the mattress 10 when a portion of the rotating ball 2130 protrudes from the frame 2110, and as the insertion groove 2111 is formed in a spherical shape whose upper portion is cut away, an upper portion of the rotating ball 2130 formed in a spherical shape may protrude from the frame 2110.

The rotating ball 2130 is rotatable by 360° while being inserted into the insertion groove 2111, and the mattress 10 may be rotated or moved in various directions through the rotating ball 2130 rotatable by 360°.

According to an embodiment of the present disclosure, the rotating ball 2130 is formed in a spherical shape, and the insertion groove 2111 may be formed in a spherical shape whose upper portion is partially cut out, but the present disclosure is not limited thereto. As long as the rotating ball 2130 can be rotated while partially protruding above the frame 2110, each of the rotating ball 2130 and the insertion groove 2111 may be formed in various shapes.

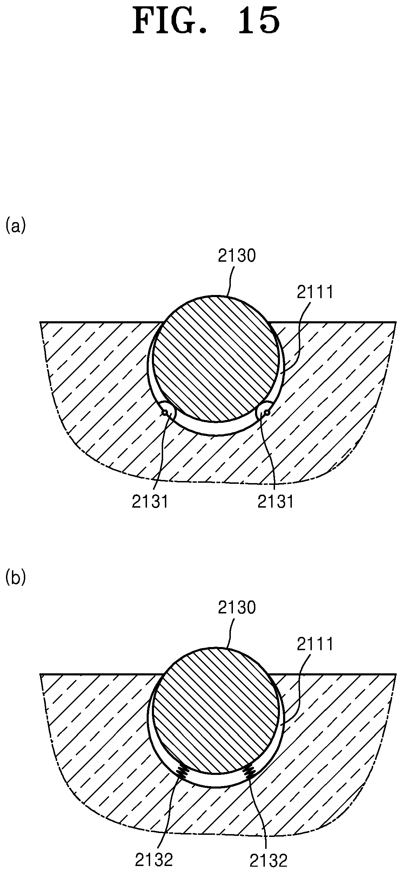

Referring to FIG. 15A, a roller 2131 in contact with the rotating ball 2130 may be provided on an inner circumferential surface of the insertion groove 2111. The roller 2131 may be used to reduce the frictional force between the rotating ball 2130 and the insertion groove 2111, and may be provided between the rotating ball 2130 and the insertion groove 2111.

When the mattress 10 comes into contact with the rotating ball 2130, the rotating ball 2130 is pressed by the weight of the mattress 10, and thus, the rotating ball 2130 may be brought into contact with the inner circumferential surface of the insertion groove 2111.

When the rotating ball 2130 is brought into contact with the inner circumferential surface of the insertion groove 2111, the rotation of the rotating ball 2130 may be restricted by the frictional force generated between the rotating ball 2130 and the inner circumferential surface of the insertion groove 2111.

The roller 2131 is provided to prevent such a phenomenon, and may reduce the frictional force generated between the rotating ball 2130 and the inner circumferential surface of the insertion groove 2111 by having the roller 2131 rotatable around one axis between the rotating ball 2130 and the inner circumferential surface of the insertion groove 2111.

Referring to FIG. 15B, a spring 2132 may also be provided between the insertion groove 2111 and the rotating ball 2130. The spring 2132 may push the rotating ball 2130 to the outside when the rotating ball 2130 is pressed by the mattress 10.

As described above, the rotation of the rotating ball 2130 may be restricted by the frictional force generated between the rotating ball 2130 and the inner circumferential surface of the insertion groove 2111. However, when the spring 2132 is provided between the rotating ball 2130 and the inner circumferential surface of the insertion groove 2111, a contact area between the rotating ball 2130 and the insertion groove 2111 may be reduced by an elastic restoring force of the spring 2132.

Specifically, as the spring 2132 pushes up the rotating ball 2130 in an outward direction (an upward protruding direction of the frame 2110), the contact area between the rotating ball 2130 and the insertion groove 2111 may be reduced, and accordingly, the frictional force generated between the rotating ball 2130 and the inner circumferential surface of the insertion groove 2111 may be reduced.

Referring to FIG. 13, the plurality of rotating balls 2130 provided in the frame 2110 may be spaced apart from each other at an interval of 30 cm to 40 cm. When the interval of the plurality of rotating balls 2130 is too large (greater than 40 cm), the contact area between the mattress 10 and the frame 2110 may be increased due to the weight of the mattress 10.

When the contact area between the mattress 10 and the frame 2110 is increased, the frictional force between the mattress 10 and the frame 2110 may increase, and thus the mattress 10 may be difficult to rotate. Thus, the interval between the plurality of rotating balls 2130 provided in the frame 2110 may be less than 40 cm.

On the contrary, when the interval between the plurality of rotating balls 2130 is too small (less than 30 cm), an excessive number of the rotating balls 2130 are formed in the frame 2110, and thus, it is difficult to adjust a rotation direction of the mattress 10. Thus, the interval between the plurality of rotating balls 2130 provided in the frame 2110 may be greater than 30 cm.

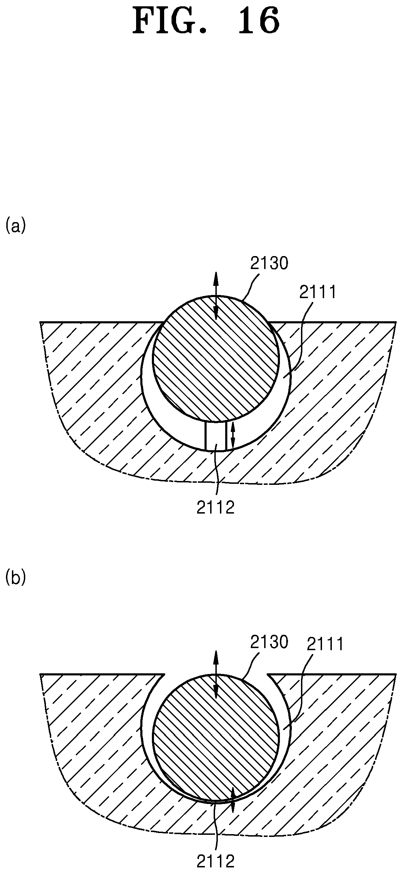

Referring to FIGS. 16A and 16B, the rotating ball 2130 may be installed to be raisable and lowerable inside the insertion groove 2111. When the edge of the mattress 10 is being processed by operating the processing part 2120, it is preferable that the rotating ball 2130 is not in contact with the mattress 10.

When the rotating ball 2130 is in contact with the mattress 10 when the edge of the mattress 10 is being processed through the processing part 2120, the operation of the processing part 2120 may be affected by the rotation of the rotating ball 2130.

Thus, when the edge of the mattress 10 is being processed through the processing part 2120, it is preferable that the rotating ball 2130 is not in contact with the mattress 10. To this end, the rotating ball 2130 may be installed to be raisable and lowerable inside the insertion groove 2111.

Specifically, the rotating ball 2130 may be installed such that a portion of the rotating ball 2130 does not protrude above the frame 2110 when the rotating ball 2130 is lowered, and protrudes above the frame 2110 when the rotating ball 2130 is raised.

A moving part 2112 may be provided between the rotating ball 2130 and the inner circumferential surface of the insertion groove 2111 in order to raise or lower the rotating ball 2130. The moving part 2112 may move up or down the rotating ball 2130 while being in contact with one side of the rotating ball 2130, and may slidably move toward inside the insertion groove 2111.

The sewing apparatus having a sheet support using a rotating ball according to an embodiment of the present disclosure may further include a switch 2140 capable of controlling the operation of the moving part 2112. The switch 2140 is a device capable of slidably moving the moving part 2112, and the raising or lowering of the rotating ball 2130 may be controlled through the switch 2140.

A user may raise the rotating ball 2130 through the switch 2140 only when the mattress 10 is rotated, and may lower the rotating ball 2130 through the switch 2140 when the edge of the mattress 10 is processed through the processing part 2120.

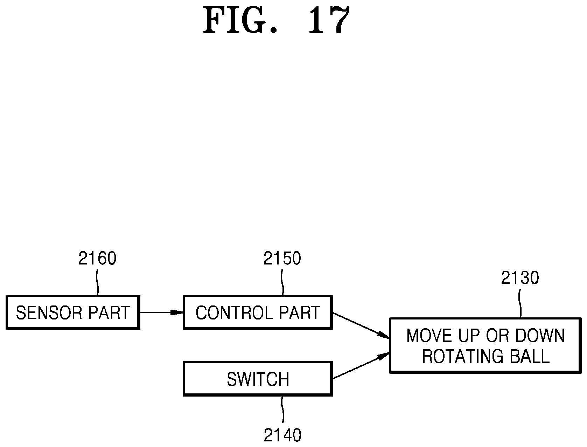

Referring to FIG. 17, the sewing apparatus having a sheet support using a rotating ball according to an embodiment of the present disclosure may operate the moving part 2112 through the switch 2140, and may also operate the moving part 2112 through a control part 2150 and a sensor part 2160.

The control part 2150 may control the operation of the moving part 2112, and may raise the rotating ball 2130 by operating the moving part 2112 when the mattress 10 is rotated as the operation of the processing part 2120 is stopped.

When an edge of the mattress 10 is being processed by operating the processing part 2120, the rotating ball 2130 may be lowered. When the rotating ball 2130 is raised when the edge of the mattress 10 is being processed through the processing part 2120, the operation of the processing part 2120 may be affected by the rotation of the rotating ball 2130.

Accordingly, the control part 2150 may control the moving part 2112 such that the rotating ball 2130 is lowered when the processing part 2120 is operated, and control the moving part 2112 such that the rotating ball 2130 is raised only when the processing part 2120 is not operated.

The control part 2150 may detect whether the mattress 10 is placed on the upper portion of the frame 2110 using the sensor part 2160, and may detect whether the mattress 10 is rotated using the sensor part 2160.

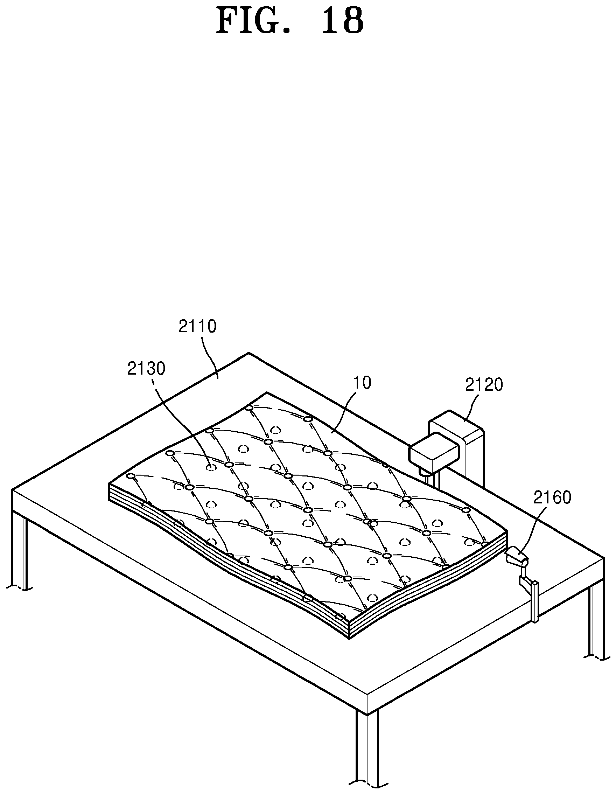

Referring to FIG. 18, the sensor part 2160 may be provided on the upper portion of the frame 2110, and whether the mattress 10 is placed or not and rotated or not may be detected by the sensor part 2160. Various sensors may be used for the sensor part 2160 as long as they can detect whether the mattress 10 is placed or not and rotated or not.

The control part 2150 may detect whether the mattress 10 is rotated using the sensor part 2160, and may also automatically raise the rotating ball 2130 only when the mattress 10 is rotated as the operation of the processing part 2120 is stopped.

Here, the control part 2150 is connected to the processing part 2120, and the control part 2150 may recognize whether the processing part 2120 operates. In addition, the control part 2150 may be disposed at various points as long as the operation of the moving part 2112 can be controlled while receiving signals of the sensor part 2160.

In the sewing apparatus having a sheet support using a rotating ball according to an embodiment of the present disclosure, an alignment bar 2170 protruding above the frame 2110 and being in contact with one side of the mattress 10 may be provided in the frame 2110.

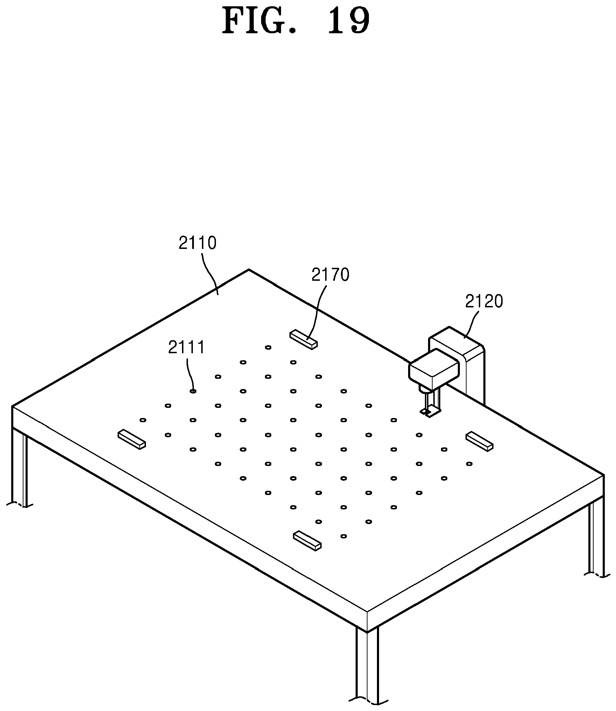

Referring to FIG. 19, the alignment bar 2170 is disposed on a line extending from the processing part 2120, protrudes above the frame 2110, and is capable of supporting the mattress 10 while being in contact with one side of the mattress 10.

The alignment bar 2170 may align one side of the mattress 10 so that the mattress 10 may pass in a straight line without shaking when passing through the processing part 2120, and due to the alignment bar 2170, a target edge point of the mattress 10 may pass through the processing part 2120 without shaking.

The alignment bar 2170 is described as being disposed on the line extending from the processing part 2120, but is not limited thereto, and may be disposed at various points as long as it can align the mattress 10 while being in contact with one side of the mattress 10.

The alignment bar 2170 may also be installed to be raisable and lowerable in the frame 2110. As described above, the mattress 10 should rotate after one side edge thereof is processed.

However, when the alignment bar 2170 protrudes above the frame 2110, the rotation of the sheet may be hindered by the alignment bar 2170. Thus, when the mattress 10 rotates, it is preferable that the alignment bar 2170 does not protrude above the frame 2110.

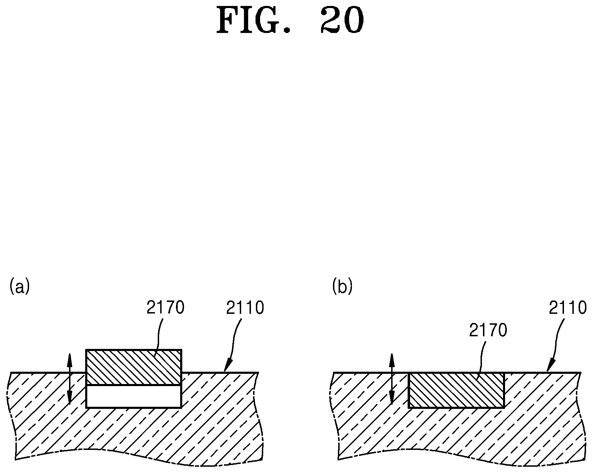

To this end, referring to FIGS. 20A and 20B, the alignment bar 2170 is raisable or lowerable in an inner direction of the frame 2110, so that the alignment bar 2170 protrudes above the frame 2110 or does not protrude above the frame 2110.

Here, the operation of the alignment bar 2170 may be controlled through the control part 2150. The control part 2150 controls the alignment bar 2170 to be lowered in the inner direction of the frame 2110 when the rotating ball 2130 is raised by the moving part 2112.

Since the phrase "the rotating ball 2130 is raised" indicates that the mattress 10 rotates, when the rotating ball 2130 is raised, the control part 2150 controls the alignment bar 2170 to be lowered in the inner direction of the frame 2110.

Thereafter, when the rotating ball 2130 is lowered, the control part 2150 may align the mattress 10 while raising the alignment bar 2170 so as to protrude above the frame 2110.

The sewing apparatus having a sheet support using a rotating ball according to an embodiment of the present disclosure has the following effects.

In the sewing apparatus having a sheet support using a rotating ball according to an embodiment of the present disclosure, the rotating ball 2130 is provided in the frame 2110 of the processing device configured to process an edge of the mattress 10, so that the edge of the mattress 10 can be processed while easily rotating and moving the mattress 10.

In particular, in the sewing apparatus having a sheet support using a rotating ball according to an embodiment of the present disclosure, the raising or lowering of the rotating ball 2130 is controlled through the control part 2150, so that the rotating ball 2130 can be controlled to be raised only when the mattress 10 is rotated.

In addition, in the sewing apparatus having a sheet support using a rotating ball according to an embodiment of the present disclosure, an edge point of the mattress 10 to be processed can accurately pass through the processing part 2120 by the alignment bar 2170, and the alignment bar 2170 is controlled through the control part 2150, so that the mattress 10 can rotate without being hindered by the alignment bar 2170 during rotation.

Hereinafter, a sewing apparatus having a sheet support using an air compressor according to an embodiment of the present disclosure will be described in detail with reference to the accompanying drawings.

The present disclosure relates to a sewing apparatus having a sheet support using an air compressor, and relates to a sewing apparatus having a sheet support using an air compressor, capable of processing an edge of a sheet by easily rotating the sheet by providing an air compressor in a frame of a processing device configured to process an edge of a sheet.

A sheet processed by the sewing apparatus having a sheet support using an air compressor according to an embodiment of the present disclosure may be a sheet placed on the mattress, but the present disclosure is not limited thereto, and the sheet may be a mattress.

In addition, a processing operation performed in the sewing apparatus having a sheet support using an air compressor according to an embodiment of the present disclosure may be a sewing operation through a sewing apparatus such as a sewing machine, but is not limited thereto, and may include various operations as long as they are operations that process an edge of a sheet or a mattress.

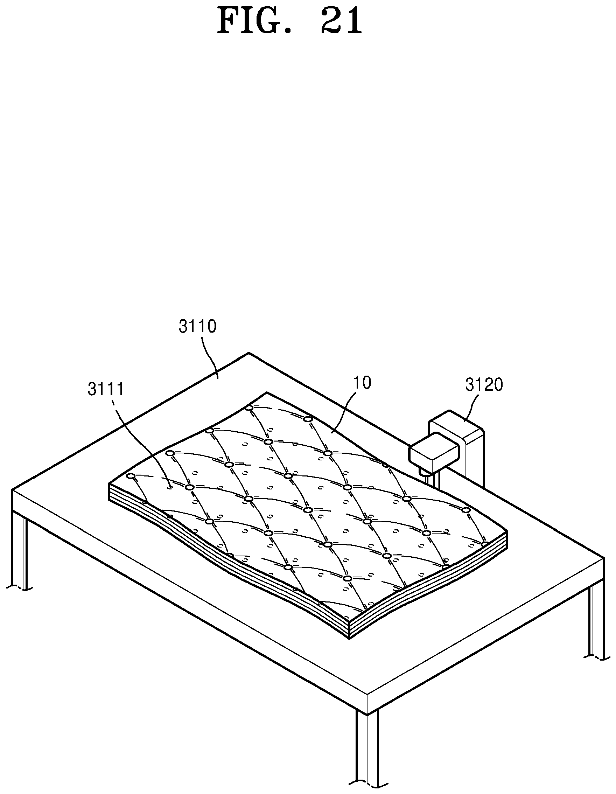

Referring to FIG. 21, the sewing apparatus having a sheet support using an air compressor according to an embodiment of the present disclosure includes a frame 3110, a processing part 3120, and an air compressor 3130.

The frame 3110 is a work bench on which a mattress 10 is placed, and may be formed of a frame in which a plate is installed in an upper portion thereof so that an edge of the mattress 10 may be processed thereon. The frame 3110 may be formed in various shapes as long as the mattress 10 is placed and processed thereon.

The processing part 3120 may be disposed on one side of the frame 3110, and may process an edge of the mattress 10. The processing part 3120 is a device capable of sewing an edge of the mattress 10, and may be a device such as a sewing machine.

However, the processing part 3120 is not limited to the sewing machine, and may include various devices as long as they are devices capable of processing an edge of the mattress 10. For example, the processing part 3120 may be a device configured to seal and sew an edge of the mattress 10.

An edge of the mattress 10 may be processed while passing through the processing part 3120. The mattress 10 is formed in a rectangular shape, and after one edge of the mattress 10 passes through the processing part 3120, the mattress 10 is rotated so that another edge of the mattress 10 is processed.

A configuration capable of rotating a sheet is not provided in a conventional sheet processing device. Since the sheet has a large volume and a significant weight, through the conventional sheet processing apparatus, it is difficult to process the sheet while rotating the sheet.

In order to solve the above problem, the sewing apparatus having a sheet support using an air compressor according to an embodiment of the present disclosure may use the air compressor 3130.

The air compressor 3130 is provided below the frame 3110 and is capable of spraying air into the frame 3110. The air compressor 3130 may include various devices as long as they are devices capable of spraying air into the frame 3110,

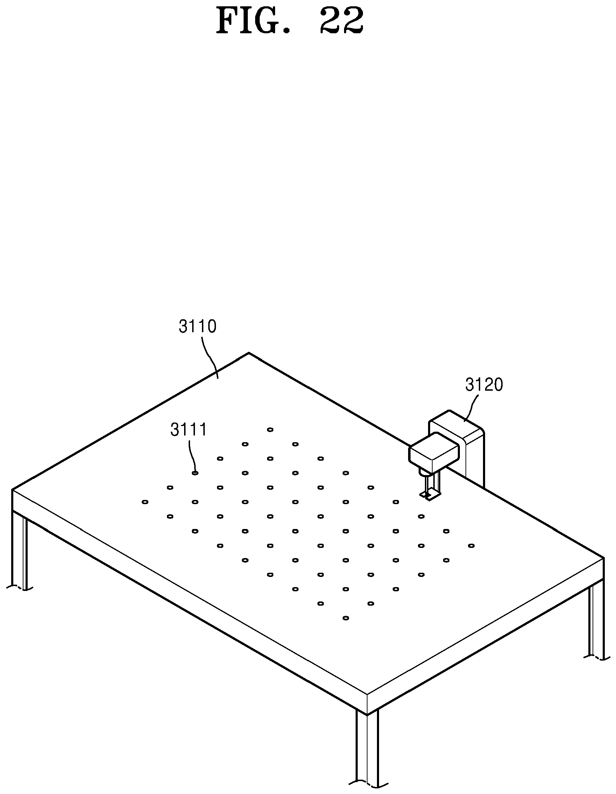

Referring to FIG. 22, the frame 3110 may include a plurality of air spraying holes 3111 through which air sprayed from the air compressor 3130 may be discharged to the outside. The plurality of air spraying holes 3111 are provided to be spaced apart from each other, and air is sprayed from each of the plurality of air spraying holes 3111.

The mattress 10 may be placed on an upper portion of the air spraying hole 3111, and may receive pressure of the air sprayed from the air spraying hole 3111. The mattress 10 may be raised upwards by the pressure of the air sprayed from the air spraying hole 3111.

The mattress 10 is difficult to rotate due to a frictional force generated between the mattress 10 and the frame 3110. However, when the mattress 10 is raised by the air sprayed from the air compressor 3130, the frictional force generated between the mattress 10 and the frame 3110 is reduced, so that the mattress 10 may be easily rotated.

Here, the mattress 10 is slightly raised by the air sprayed from the air compressor 3130, and the mattress 10 may be raised only to the extent that the mattress 10 and the frame 3110 are not in contact with each other.

Referring to FIG. 22, the plurality of air spraying holes 3111 provided in the frame 3110 may be spaced apart from each other at an interval of 40 cm to 50 cm. When the interval of the plurality of air spraying holes 3111 is to large (greater than 50 cm), sufficient force may not be supplied to the mattress 10.

In addition, when the interval between the plurality of air spraying holes 3111 is too large (greater than 50 cm), a separation distance between points at which the mattress 10 receives force by air pressure increases, and thus the balance of the mattress 10 may be destroyed. Accordingly, the separation distance between the air spraying holes 3111 may less than 50 cm.

On the contrary, when the interval between the plurality of air spraying holes 3111 is too small (less than 40 cm), excessive force may be applied to the mattress 10. When the mattress 10 is raised only to the extent that the frictional force is not generated between the mattress 10 and the frame 3110, the mattress 10 is easily rotated.

Accordingly, it is not necessary to apply excessive force to the mattress 10, and air may be sprayed so that the mattress 10 is raised only to the extent that the frictional force is not generated between the mattress 10 and the frame 3110. Thus, the interval between the plurality of air spraying holes 3111 may be greater than 40 cm.

The sewing apparatus having a sheet support using an air compressor according to an embodiment of the present disclosure may further include a switch 3140 capable of controlling the operation of the air compressor 3130. The switch 3140 is a device capable of turning the air compressor 3130 on/off, and the user may operate the air compressor 3130 only when the mattress 10 is rotated.

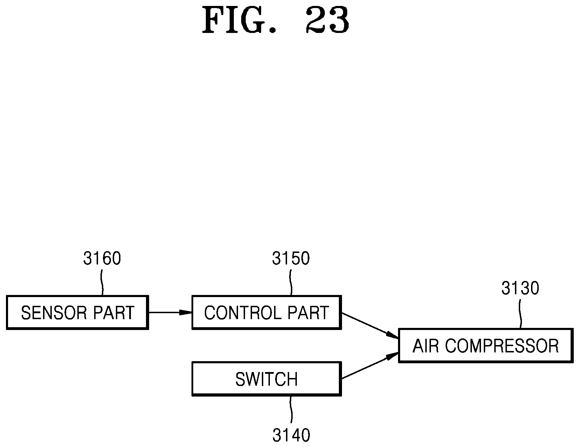

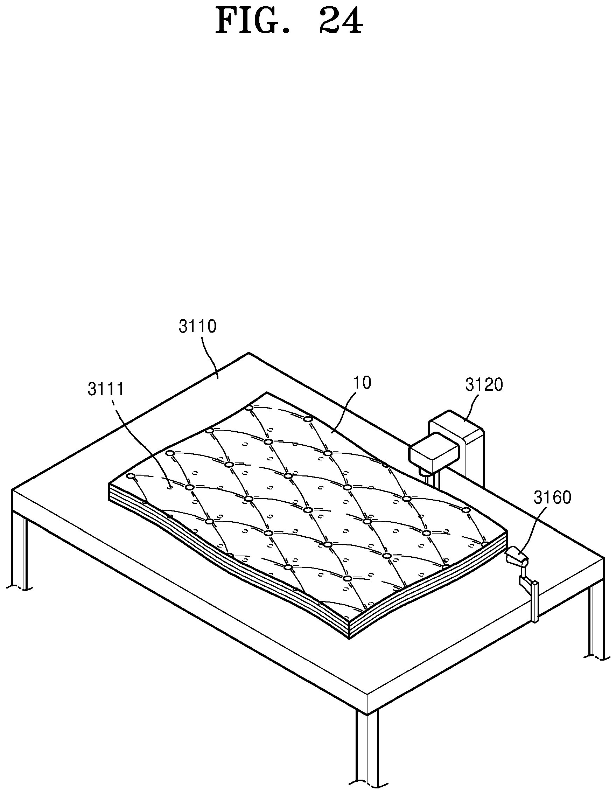

Referring to FIGS. 23 and 24, the sewing apparatus having a sheet support using an air compressor according to an embodiment of the present disclosure may further include a control part 3150 configured to control air spraying from the air compressor 3130, and a sensor part 3160 capable of detecting whether the mattress 10 is placed on the frame 3110.

Referring to FIG. 23, the sewing apparatus having a sheet support using an air compressor according to an embodiment of the present disclosure may be operated through the switch 3140, and may also be operated through the control part 3150 and the sensor part 3160.

The control part 3150 may control the air spraying from the air compressor 3130, and the control part 3150 may control the air compressor 3130 to spray air into the frame 3110 when the operation of the processing part 3120 is stopped and the mattress 10 is rotated.

When an edge of the mattress 10 is processed by operating the processing part 3120, the operation of the air compressor 3130 may be stopped. When air is sprayed from the air compressor 3130 when the edge of the mattress 10 is processed through the processing part 3120, this may affect the operation of the processing part 3120.

Accordingly, the control part 3150 may control the air compressor 3130 so that air is not sprayed from the air compressor 3130 when the processing part 3120 is operated, and air is sprayed from the air compressor 3130 only when the processing part 3120 is not operated.

The control part 3150 may detect that the mattress 10 is placed on the upper portion of the frame 3110 using the sensor part 3160. Various sensors may be used for the sensor part 3160 as long as they can detect whether the mattress 10 is placed on the frame 3110.

The control part 3150 may control air to be sprayed from the air compressor 3130 only when the sensor part 3160 detects that the mattress 10 is placed on the frame 3110.

The sensor part 3160 may also detect whether the mattress 10 rotates. Referring to FIG. 24, the sensor part 3160 may be provided on the upper portion of the frame 3110, and whether the mattress 10 is rotated or not may be detected by the sensor part 3160. Various sensors may be used for the sensor part 3160 as long as they can detect whether the mattress 10 is rotated or not.

The control part 3150 may detect whether the mattress 10 is rotated using the sensor part 3160, and may also control the air compressor 3130 to automatically spray air when the mattress 10 is rotated as the operation of the processing part 3120 is stopped.

Here, the control part 3150 is connected to the processing part 3120, and the control part 3150 may recognize whether the processing part 3120 operates. In addition, the control part 3150 may be disposed at various points as long as the operation of the air compressor 3130 can be controlled while receiving signals of the sensor part 3160.

The control part 3150 may also control the air compressor 3130 such that the pressure of the air sprayed from each of the plurality of air spraying holes 3111 is different from each other. As described above, the plurality of air spraying holes 3111 are provided in the frame 3110, and since the pressure of the air sprayed from each of the plurality of air spraying holes 3111 is different from each other, the mattress 10 may be easily rotated.

According to an embodiment, the control part 3150 controls such that the pressure of the air sprayed from the air spraying hole disposed in a center portion of the mattress 10 is formed to be greater than the pressure of the air sprayed from the air spraying hole disposed in a periphery of the mattress 10.

When the pressure of the air sprayed from the air spraying hole disposed in the center portion of the mattress 10 is formed to be great, a large force may be applied to a center of the mattress 10 to form a central axis around which the mattress 10 rotates. Accordingly, the mattress 10 may be easily rotated.

The control part 3150 may detect the center portion of the mattress 10 using the sensor part 3160. The sensor part 3160 may detect a shape of the mattress 10, and as the sensor part 3160 detects the shape of the mattress 10, the control part 3150 may recognize a center point of the mattress 10.

Here, the center portion of the mattress 10 may be an inside of a circle having a diameter of 50 cm to 200 cm with respect to the center point of the mattress 10, and the periphery of the mattress 10 may be a portion other than the center portion of the mattress 10.

The control part 3150 may also control the air compressor 3130 such that air is sprayed only from some of the plurality of air spraying holes 3111. The control part 3150 may control such that air is sprayed only from some of the plurality of air spraying holes 3111 according to a direction in which the mattress 10 rotates, and thus, the mattress 10 may be easily rotated.

In the sewing apparatus having a sheet support using an air compressor according to an embodiment of the present disclosure, an alignment bar 3170 protruding above the frame 3110 and being in contact with one side of the mattress 10 may be provided in the frame 3110.

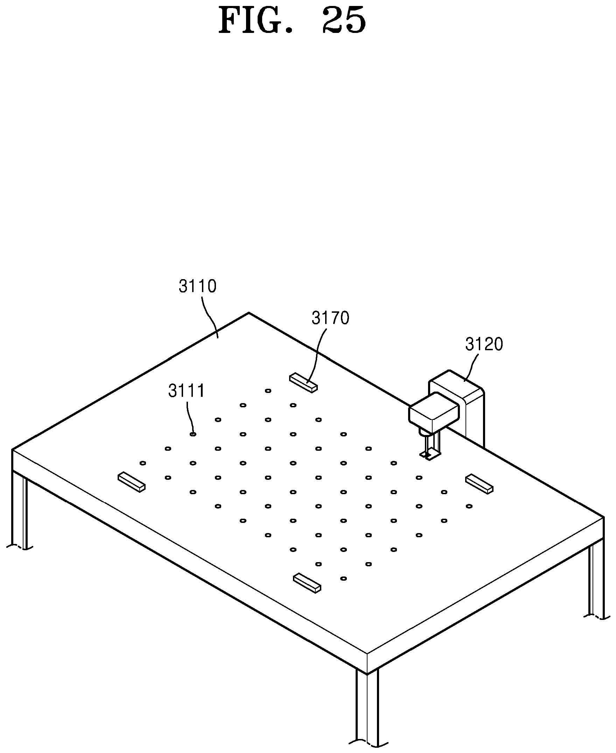

Referring to FIG. 25, the alignment bar 3170 is disposed on a line extending from the processing part 3120, protrudes above the frame 3110, and is capable of supporting the mattress 10 while being in contact with one side of the mattress 10.

The alignment bar 3170 may align one side of the mattress 10 so that the mattress 10 may pass in a straight line without shaking when passing through the processing part 3120, and due to the alignment bar 3170, a target edge point of the mattress 10 may pass through the processing part 3120 without shaking.

The alignment bar 3170 is described as being disposed on the line extending from the processing part 3120, but is not limited thereto, and may be disposed at various points as long as it can align the mattress 10 while being in contact with one side of the mattress 10.

The alignment bar 3170 may be installed to be raisable and lowerable in the frame 3110. As described above, the mattress 10 should rotate after one side edge thereof is processed.

However, when the alignment bar 3170 protrudes above the frame 3110, the rotation of the sheet may be hindered by the alignment bar 3170. Thus, when the mattress 10 rotates, it is preferable that the alignment bar 3170 does not protrude above the frame 3110.

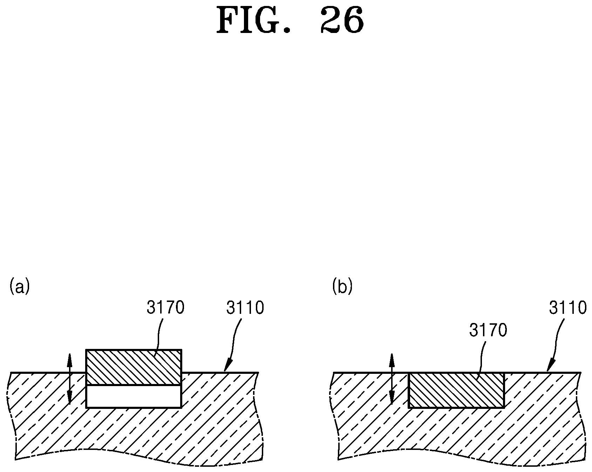

To this end, referring to FIGS. 26A and 26B, the alignment bar 3170 is raisable or lowerable in an inner direction of the frame 3110, so that the alignment bar 3170 protrudes above the frame 3110 or does not protrude above the frame 3110.

Here, the operation of the alignment bar 3170 may be controlled through the control part 3150. When air is sprayed into the frame 3110 from the air compressor 3130, the control part 3150 controls the alignment bar 3170 to be lowered in the inner direction of the frame 3110.

Since the phrase "air is sprayed from the air compressor 3130" indicates that the mattress 10 rotates, when air is sprayed into the frame 3110 from the air compressor 3130, the control part 3150 control the alignment bar 3170 to be lowered in the inner direction of the frame 3110.

Thereafter, when air is not sprayed from the air compressor 3130, the control part 3150 controls the alignment bar 3170 to be raised so that the alignment bar 3170 protrudes above the frame 3110, thereby aligning the mattress 10.

The sewing apparatus having a sheet support using an air compressor according to an embodiment of the present disclosure has the following effects.

The sewing apparatus having a sheet support using an air compressor according to an embodiment of the present disclosure includes the air compressor 3130 configured to spray air into the frame 3110 of the processing device that processes an edge of the mattress 10, so that the edge of the mattress 10 can be processed while easily rotating and moving the mattress 10.

In particular, in the sewing apparatus having a sheet support using an air compressor according to an embodiment of the present disclosure, the air compressor 3130 can be controlled by the control part 3150, and an spraying pressure or spraying point of each of the plurality of air spraying holes 3111 can be controlled, so that the mattress 10 can easily be rotated.

In addition, in the sewing apparatus having a sheet support using an air compressor according to an embodiment of the present disclosure, an edge point of the mattress 10 to be processed may accurately pass through the processing part 3120 by the alignment bar 3170, and the alignment bar 3170 is controlled through the control part 3150, so that the mattress 10 can rotate without being hindered by the alignment bar 3170 during rotation.

As set forth above, exemplary embodiments have been disclosed in the accompanying drawings and the specification. Herein, specific terms have been used, but are just used for the purpose of describing the present disclosure and are not used for qualifying the meaning or limiting the scope of the present disclosure, which is disclosed in the appended claims.

### Industrial Applicability

According to an embodiment of the present disclosure, provided is an automatic mattress sterilization apparatus. In addition, the embodiments of the present disclosure are applicable to a mattress processing apparatus such as an apparatus for processing an edge of a mattress.

## Claims

1. An automatic mattress sterilization apparatus configured to sterilize a mattress (10), comprising:
a frame (110) extending in one direction on which the mattress (10) is configured to be placed;
a sterilization part (120) coupled to an upper portion of the frame (110) and having an inner space through which the mattress (10) is configured to pass; and
a plurality of moving rollers (130) provided in the frame (110) and configured to move the mattress (10) placed on the frame(110) ,
wherein the mattress (10) is configured to be moved from one side of the sterilization part (120) to the other side of the sterilization part (120) via the inner space of the sterilization part (120) by the plurality of moving rollers (130),
further comprising a control part (150) configured to control a rotation of each of the moving rollers (131),
wherein the control part (150) controls the moving roller (131) to rotate repeatedly in forward and reverse directions so that vibration is applied to the mattress (10), when the mattress (10) is placed inside the sterilization part (120), and
the number of forward rotations of the moving roller (131) is greater than the number of reverse rotations of the moving roller (131).

2. The automatic mattress sterilization apparatus of claim 1, wherein an ultraviolet lamp configured to sterilize the mattress is provided inside the sterilization part.

3. The automatic mattress sterilization apparatus of claim 1, wherein a gas spraying part configured to spray high-pressure gas onto the mattress is provided in the sterilization part.

4. The automatic mattress sterilization apparatus of claim 1, wherein
one side and the other side of the inner space communicate with the outside so that the mattress is moved therethrough,
an inlet part configured to open and close one side of the inner space is provided at one side of the inner space, and
an outlet part configured to open and close the other side of the inner space is provided at the other side of the inner space.

5. The automatic mattress sterilization apparatus of claim 4, wherein
a first sensor part configured to detect a distance between the inlet part of the sterilization part and one end of the mattress is provided at one side of the sterilization part, and
a second sensor part configured to detect a distance between one end of the mattress moving in the inner space and the outlet part of the sterilization part is provided inside the sterilization part.

6. The automatic mattress sterilization apparatus of claim 5, further comprising a control part configured to control the opening and closing of each of the inlet part and the outlet part,
wherein the control part controls each of the inlet part and the outlet part to be open when the distance detected by each of the first sensor part and the second sensor part is less than or equal to a designated value.

7. The automatic mattress sterilization apparatus of claim 1, wherein a vibration application part configured to apply vibration to the mattress is provided in the sterilization part.

## Patentansprüche

1. Automatisches Matratzensterilisationsgerät, das dazu konfiguriert ist, eine Matratze (10) zu sterilisieren, umfassend:
ein Gestell (110), das sich in einer Richtung erstreckt, wobei die Matratze (10) dazu konfiguriert ist, darauf gelegt zu werden;
einen Sterilisationsteil (120), der mit einem oberen Abschnitt des Gestells (110) gekoppelt ist und einen Innenraum aufweist, wobei die Matratze (10) dazu konfiguriert ist, durch diesen hindurch zu gehen; und
eine Vielzahl von beweglichen Rollen (130), die in dem Gestell (110) bereitgestellt werden und dazu konfiguriert sind, die Matratze (10), die auf dem Gestell (110) liegt, zu bewegen;
wobei die Matratze (10) dazu konfiguriert ist, von einer Seite des Sterilisationsteils (120) zu der anderen Seite des Sterilisationsteils (120) über den Innenraum des Sterilisationsteils (120) durch die Vielzahl von beweglichen Rollen (130) bewegt zu werden,
ferner umfassend einen Steuerteil (150), der dazu konfiguriert ist, eine Drehung jeder der beweglichen Rollen (131) zu steuern,
wobei der Steuerteil (150) die bewegliche Rolle (131) steuert, damit sie sich wiederholt in die Vorwärts- und Rückwärtsrichtungen dreht, so dass eine Vibration auf die Matratze (10) ausgeübt wird, wenn die Matratze (10) in den Sterilisationsteil (120) hinein gelegt wird; und
die Anzahl von Vorwärtsdrehungen der beweglichen Rolle (131) größer als die Anzahl von Rückwärtsdrehungen der beweglichen Rolle (131) ist.

2. Automatisches Matratzensterilisationsgerät nach Anspruch 1, wobei eine ultraviolette Leuchte, die dazu konfiguriert ist, die Matratze zu sterilisieren, im Innern des Sterilisationsteils bereitgestellt wird.

3. Automatisches Matratzensterilisationsgerät nach Anspruch 1, wobei ein Gassprühteil, der dazu konfiguriert ist, Hochdruckgas auf die Matratze zu sprühen, in dem Sterilisationsteil bereitgestellt wird.

4. Automatisches Matratzensterilisationsgerät nach Anspruch 1, wobei
eine Seite und die andere Seite des Innenraums mit der Außenseite in Verbindung stehen, so dass die Matratze durch diese hindurch bewegt wird,
ein Einlassteil, der dazu konfiguriert ist, eine Seite des Innenraums zu öffnen und zu schließen, auf einer Seite des Innenraums bereitgestellt wird, und
ein Auslassteil, der dazu konfiguriert ist, die andere Seite des Innenraums zu öffnen und zu schließen, auf der anderen Seite des Innenraums bereitgestellt wird.

5. Automatisches Matratzensterilisationsgerät nach Anspruch 4, wobei
ein erster Sensorteil, der dazu konfiguriert ist, einen Abstand zwischen dem Einlassteil des Sterilisationsteils und einem Ende der Matratze zu detektieren, auf einer Seite des Sterilisationsteils bereitgestellt wird, und
ein zweiter Sensorteil, der dazu konfiguriert ist, einen Abstand zwischen einem Ende der Matratze, die sich in dem Innenraum bewegt, und dem Auslassteil des Sterilisationsteils zu detektieren, im Innern des Sterilisationsteils bereitgestellt wird.

6. Automatisches Matratzensterilisationsgerät nach Anspruch 5, ferner umfassend einen Steuerteil, der dazu konfiguriert ist, das Öffnen und Schließen jedes von dem Einlassteil und dem Auslassteil zu steuern,
wobei der Steuerteil jeden von dem Einlassteil und dem Auslassteil steuert, damit er offen ist, wenn der Abstand, der von jedem von dem ersten Sensorteil und dem zweiten Sensorteil detektiert wird, kleiner oder gleich einem bestimmten Wert ist.

7. Automatisches Matratzensterilisationsgerät nach Anspruch 1, wobei ein Vibrationsanwendungsteil, der dazu konfiguriert ist, eine Vibration auf die Matratze anzuwenden, in dem Sterilisationsteil bereitgestellt wird.

## Revendications

1. Appareil de stérilisation automatique de matelas configuré pour stériliser un matelas (10), comprenant :
un cadre (110) s'étendant dans une direction et sur lequel le matelas (10) est configuré pour être placé ;
une partie stérilisation (120) couplée à une partie supérieure du cadre (110) et présentant un espace intérieur à travers lequel le matelas (10) est configuré pour passer ; et
une pluralité de rouleaux mobiles (130) prévus dans le cadre (110) et configurés pour déplacer le matelas (10) placé sur le cadre (110),
le matelas (10) étant configuré pour être déplacé d'un côté de la partie stérilisation (120) à l'autre côté de la partie stérilisation (120) via l'espace intérieur de la partie stérilisation (120) par la pluralité de rouleaux mobiles (130),
comprenant en outre une partie commande (150) configurée pour commander une rotation de chacun des rouleaux mobiles (131),
la partie commande (150) commandant le rouleau mobile (131) pour le faire tourner de manière répétée dans des directions avant et arrière de sorte que des vibrations sont appliquées au matelas (10) lorsque le matelas (10) est placé à l'intérieur de la partie stérilisation (120), et
le nombre de rotations vers l'avant du rouleau mobile (131) étant supérieur au nombre de rotations vers l'arrière du rouleau mobile (131).

2. Appareil de stérilisation automatique de matelas selon la revendication 1, dans lequel une lampe à ultraviolets configurée pour stériliser le matelas est prévue à l'intérieur de la partie stérilisation.

3. Appareil de stérilisation automatique de matelas selon la revendication 1, dans lequel une partie pulvérisation de gaz configurée pour pulvériser un gaz à haute pression sur le matelas est prévue dans la partie stérilisation.

4. Appareil de stérilisation automatique de matelas selon la revendication 1, dans lequel
un côté et l'autre côté de l'espace intérieur communiquent avec l'extérieur, de sorte que le matelas est déplacé à travers celui-ci,
une partie entrée configurée pour ouvrir et fermer un côté de l'espace intérieur est prévue sur un côté de l'espace intérieur, et
une partie sortie configurée pour ouvrir et fermer l'autre côté de l'espace intérieur est prévue sur l'autre côté de l'espace intérieur.

5. Appareil de stérilisation automatique de matelas selon la revendication 4, dans lequel
une première partie capteur est configurée pour détecter une distance entre la partie entrée de la partie stérilisation et une extrémité du matelas est prévue sur un côté de la partie stérilisation, et
une seconde partie capteur configurée pour détecter une distance entre une extrémité du matelas se déplaçant dans l'espace intérieur et la partie sortie de la partie de stérilisation est prévue à l'intérieur de la partie stérilisation.

6. Appareil de stérilisation automatique de matelas selon la revendication 5, comprenant en outre une partie commande configurée pour commander l'ouverture et la fermeture de chacune de la partie entrée et de la partie sortie,
la partie commande commandant que chacune des parties d'entrée et de sortie soit ouverte lorsque la distance détectée par chacune des première et seconde parties capteurs est inférieure ou égale à une valeur désignée.

7. Appareil de stérilisation automatique de matelas selon la revendication 1, dans lequel une partie application de vibrations configurée pour appliquer des vibrations au matelas est prévue dans la partie stérilisation.
